# EUROPEAN PATENT APPLICATION

(11) **EP 4 397 776 A2**
(43) Date of publication of application: **10.07.2024**
(21) Application number: 24163844.4
(22) Date of filing: 14.08.2019
(51) Int. Cl.: C12Q 1/6886

(54) **METHOD FOR CLASSIFYING A PATIENT'S RESPONSIVENESS TO IMMUNE CHECKPOINT INHIBITOR THERAPY**

(62) Divisional of application: 19191783.0
(71) Applicant: Eberhard Karls Universität Tübingen (Medizinische Fakultät), 72074 Tübingen (DE)
(72) Inventor: Röcken, Martin, 72074 Tübingen (DE); Rieß, Olaf, 72070 Tübingen (DE); Hilke, Franz Joachim, 72074 Tübingen (DE); Brenner, Ellen, 72074 Tübingen (DE)
(74) Representative: Witte, Weller & Partner Patentanwälte mbB

(57) **Abstract**

The present invention relates to a method *ex vivo* for classifying a patient in need as non-responder or responder to immune checkpoint inhibitor therapy, and the use of a gene set for classifying a patient in need as non-responder or responder to immune checkpoint inhibitor therapy.

## Description

The present invention relates to a method ex vivo for classifying a patient in need as non-responder or responder to immune checkpoint inhibitor therapy, and the use of a gene set for classifying a patient in need as non-responder or responder to immune checkpoint inhibitor therapy.

### FIELD OF THE INVENTION

The present invention relates to the field of companion diagnostics, more particular to classifying a patient as non-responder or responder to immune checkpoint inhibitor therapy by means of molecular biomarker.

### BACKGROUND OF THE INVENTION

Checkpoint inhibitor therapy, also referred to as checkpoint blockade therapy, is a form of cancer immunotherapy. The therapy targets immune checkpoints, key regulators of the immune system that stimulate or inhibit its actions, which tumors can use to protect themselves from attacks by the immune system. Checkpoint therapy can block inhibitory checkpoints, restoring immune system function.

Melanoma, also known as malignant melanoma, is a type of cancer that develops from the pigment-containing cells known as melanocytes. Melanomas typically occur in the skin, but may rarely occur primarily in lymph nodes, in the mouth, intestines, or eye.

A large part of melanoma patients are currently treated with checkpoint inhibitors, including anti-CTLA-4 monoclonal antibodies (ipilimumab and tremelimumab), toll-like receptor (TLR) agonists, CD40 agonists, anti-PD-1 (e.g. pembrolizumab, pidilizumab, and nivolumab) and PD-L1 antibodies.

Approximately 40% of all melanoma patients undergoing checkpoint inhibitor therapy show a good response to checkpoint inhibitor therapy for more than one year and are referred to as responders. However, the remaining part of patients develops a tumor progression many of within three months of immune therapy. Such patients are referred to as non-responders.

Checkpoint inhibitor therapy is expensive and time-consuming. Further, altering checkpoint inhibition can have diverse effects on most organ systems of the body including serious immunological adverse effects in up to 40 % of the patients. For these reasons, the beginning of checkpoint inhibitor therapy should be given careful consideration.

So far there is no method available in the art which allows the early, rapid and practicable identification of non-responders to checkpoint inhibitor therapy.

Therefore, there is a need in the art for the provision of a method which allows an early identification of non-responders to checkpoint inhibitor therapy, preferably before the actual therapy has been started. This would allow the avoidance of an ineffective, expensive therapy and the potential occurrence of side effects.

It is, thus, an object underlying the invention to provide such a method by means of which the disadvantages in the art can be reduced or even avoided.

The present invention satisfies these and other needs.

### SUMMARY OF THE INVENTION

The present invention provides a method ex *vivo* for classifying a patient in need as non-responder or responder to immune checkpoint inhibitor therapy, comprising the following steps:
1) providing a biological sample originating from said patient;
2) analyzing the biological sample for the presence or absence of a genetic alteration causing a modification of function in
   - cellular senescence-inducing cell cycle regulator genes, and/or
   - cellular senescence-inhibiting cell cycle regulator genes,
3) classifying the patient as
   - non-responder if in step (2) said genetic alterations are identified as present, or
   - responder if in step (2) said genetic alterations are identified as absent.

The inventors analyzed tumors in responders and non-responders in checkpoint inhibitor therapy by means of next generation sequencing (NGS). They were able to identify in the group of therapy-resistant patients, i.e. non-responders, a loss of function of cellular senescence-inducing cell cycle regulator genes, and a strong amplification of cellular senescence-inhibiting cell cycle regulator genes. Non-responders had significantly more inactivating mutations in senescence-inducing cell cycle regulator genes, and a more than 4-fold amplification of genes which accelerate the cell cycle and inhibit senescence induction.

The inventors found out that this gene patter is suitable for a prediction of patients which respond well to checkpoint inhibitor therapy, already before the therapy has been started, and is therefore proposed as "companion diagnostics" to be carried out before an actual treatment of diseases such as melanoma with checkpoint inhibitors will be initiated.

According to the invention a "patient in need" refers to any kind of living being, including a human or animal, such as a tumor patient, which may benefit from checkpoint inhibitor therapy.

A "biological sample" according to the invention refers to any biologic material containing genetic information allowing the determination of presence or absence of cell cycle regulator genes. Examples include nucleated cells such as tumor cells, tissue such as tumor tissue, etc.

"Analyzing" according to the invention refers to any method allowing the determination of presence or absence of cell cycle regulator genes in the biological sample. Examples include methods involving the sequencing of nucleic acids, such as next generation sequencing (NGS) or panel sequencing or other genetic methods.

According to the invention a "modification of function" means any kind of functional change of cell cycle regulator genes over the wild type, including a decrease, loss, increase, multiplication, and amplification of gene expression, gene copies or the activity of the corresponding gene products.

According to the invention "cellular senescence-inducing cell cycle regulator genes" refers to such genes having a key and/or control function in cellular senescence, where the activity of the products of such genes directs the cell towards cellular senescence, e.g. by inhibiting cell cycle progression. Prototypes of these genes are cyclin-dependent kinase inhibitors (CKI).

According to the invention "cellular senescence-inhibiting cell cycle regulator genes" refers to such genes having a key and/or control function in cellular senescence, where the activity of the products of such genes directs the cell through cell cycle progression, e.g. by driving the cell cycle. Prototypes of these genes are cyclin-dependent kinases (CDK).

Cellular senescence genes, including cellular senescence-inducing cell cycle regulator genes and cellular senescence-inhibiting cell cycle regulator genes, are well known in the art and can be retrieved from pertinent databases, e.g. the "CellAge" database run by Human Ageing Genomic Resources; see Tacutu, R., Craig, T., Budovsky, A., Wuttke, D., Lehmann, G., Taranukha, D., Costa, J., Fraifeld, V. E., de Magalhaes, J. P. (2013) "Human Ageing Genomic Resources: Integrated databases and tools for the biology and genetics of ageing." Nucleic Acids Research 41 (D1):D1027-D1033.

According to the invention "classifying" refers to the allocation of the patient the biological sample originates from to any of the groups of responders or non-responders.

The problem underlying the invention is herewith completely solved.

While the existence of senescence-regulating genes is known in the art it was not to be expected but surprising that such genes can serve as predictors for the responsiveness of a patient to checkpoint inhibitor therapy.

The invention also allows the conclusion that a patient who is non-responsive to checkpoint inhibitor therapy may preferably be alternatively or additionally (combination therapy) subjected to a treatment with directly senescence-inducing compounds like CDK4 inhibitors and/or CDK 6 inhibitors and/or CDK8 inhibitors.

In an embodiment of the invention said genetic alteration causing a modification of function is a
- loss-of-function mutation in cellular senescence-inducing cell cycle regulator genes, and/or
- gain-of-function mutation in cellular senescence-inhibiting cell cycle regulator genes.

The measure has the advantage that the sample is analyzed for such kinds of genetic alterations which, according to the findings of the inventors, are mainly responsible for the responsiveness of a patient to immune checkpoint inhibitor therapy. The measure therefore results in an increase of work efficiency.

According to the invention "loss-of-function mutation" refers to any kind of alteration of the nucleotide sequence of the cell cycle regulator genes which results in a restriction or even failure of the functionality and/or activity of the encoded gene product, such as a gene deletion, frame shift mutation or other function-inhibiting mutations.

According to the invention "gain-of-function mutation" refers to any kind of alteration of the nucleotide sequence of the cell cycle regulator gene including an amplification of the coding sequence in the genome which results in an increase of the functionality and/or activity of the encoded gene product.

In another embodiment of the invention said cellular senescence-inducing cell cycle regulator genes are selected from the group consisting of: *CDKN2A, CDKN2B, CDKN2C, CDKN1A, CDKN1B, RB1, TP53, STAT1, JAK1, JAK2, and JAK3.*

This measure has the advantage that such cellular senescence-inducing cell cycle regulator genes are analyzed which, according to the findings of the inventors, have particular predictive power.

*"CDKN2A",* also known as cyclin-dependent kinase Inhibitor 2A codes for two proteins, including the INK4 family member p16 (or p16^{INK4a}) and p14arf. Both act as tumor suppressors by regulating the cell cycle.

*"CDKN2B"* stands for cyclin-dependent kinase 4 inhibitor B which is also known as multiple tumor suppressor 2 (MTS-2) or p15^{INK4b}. It is a cyclin-dependent kinase inhibitor which forms a complex with CDK4 or CDK6. The inhibitor prevents the activation of the CDK kinases by cyclin D, thus the encoded protein functions as a cell growth regulator that blocks cell cycle G1 progression.

*"CDKN2C"* or cyclin-dependent kinase 4 inhibitor C is another enzyme of the INK4 family of cyclin-dependent kinase inhibitors. This protein has been shown to interact with CDK4 or CDK6, and prevent the activation of the CDK kinases, thus function as a cell growth regulator that controls cell cycle G1 progression.

*"CDKN1A",* also referred to as p21^{Cip1}, cyclin-dependent kinase inhibitor 1 or CDK-interacting protein 1, is a key cyclin-dependent kinase inhibitor (CKI) that is capable of inhibiting all cyclin/CDK complexes. It is a central molecule for the inhibition of CDK4 and CDK6 that themselves are key molecules for the induction of cellular senescence. *CDKN1A* is also strongly associated with the inhibition of CDK2, another molecule that may regulate the transcription factor E2F2.

*"CDKN1B"* stands for cyclin-dependent kinase inhibitor 1B and is also known as p27^{Kip1}. It encodes a protein which belongs to the *Cip*/*Kip* family of cyclin-dependent kinase inhibitor proteins. The encoded protein binds to and prevents the activation of cyclin E-CDK2 or cyclin D-CDK4 complexes, and thus controls the cell cycle progression at G1. Enhanced activity of *CDKN1B* and, in consequence, function activating mutations, can activate E2F2. Function-inactivating mutations can inactivate E2F. Therefore, *CDKN1B* qualifies as both cellular senescence-inducing cell cycle regulator gene and cellular senescence-inhibiting cell cycle regulator gene

*"RB1"* stands for retinoblastoma protein 1 and is a tumor suppressor protein that is dysfunctional in several major cancers. One function of Rb is to prevent excessive cell growth by inhibiting cell cycle progression until a cell is ready to divide. The hypo-phosphorylated form of *RB1* impairs cell cycle progression as it does not activate E2F2, while the phosphorylated form promotes the cell cycle progression by activating the transcription factor E2F2. That is why *RB1* qualifies as both cellular senescence-inducing cell cycle regulator gene and cellular senescence-inhibiting cell cycle regulator gene.

*"TP53"* encodes tumor protein p53 which is crucial in multicellular organisms, where it prevents cancer formation, thus, functions as a tumor suppressor. Activated p53 binds DNA and activates expression of several genes including microRNA miR-34a, WAF1/CIP1 encoding for p21 and hundreds of other down-stream genes. It can arrest growth by holding the cell cycle at the G1/S regulation point. It is a key molecule for senescence induction as it activates *CDKN1A.*

*"STAT1"* means 'signal transducer and activator of transcription 1' and encodes a transcription factor. In addition and importantly, the STAT1 protein directly interacts with cyclin D1/Cdk4 and mediates cell cycle arrest. STAT1 transmits the signals of type I and type II interferons and thus is essential for the activation of the MDM2/4-p21-CDK4/6-Rb1 and the p16/p14-CDK4/6-Rb1 senescence pathways.

*"JAK1"* stands for 'Janus kinase 1' and encodes a tyrosine kinase protein essential for signaling for certain type I and type II cytokines namely type I and type II interferons, and the interferon mediated activation of STAT1 what activates the MDM2/4-p21-CDK4/6-Rb1 and the p16/p14-CDK4/6-Rb1 senescence pathways. Loss of Jak1 is lethal in neonatal mice. It is known that the loss of the expression of JAK1 in cancer cells enables tumor cells to escape from tumor cell killing and to metastasize to other parts of the body.

*"JAK2"* stands for 'Janus kinase 2' and encodes a non-receptor tyrosine kinase which has been implicated in signaling by members of the type II cytokine receptor family and other signaling molecules. Loss of Jak2 is lethal by embryonic day 12 in mice. It transmits interferon mediated activation of STAT1 what activates the MDM2/4-p21-CDK4/6-Rb1 and the p16/p14-CDK4/6-Rb1 senescence pathways.

*"JAK3"* stands for 'Janus kinase 3'. It codes for a tyrosine kinase that is specifically associated with cytokine receptors. JAK3 is involved in signal transduction by receptors that employ the common gamma chain (γc) of the type I cytokine receptor family. It transmits interferon mediated activation of STAT1 what activates the MDM2/4-p21-CDK4/6-Rb1 and the p16/p14-CDK4/6-Rb1 senescence pathways.

According to the invention the human variants of above genes and gene products are preferred.

In another embodiment of the invention said cellular senescence-inhibiting cell cycle regulator genes are selected from the group consisting of: *CCND1, CCND2, CCND3, CDK4, CDK6, CDKN1B, CCNE1, RB1, E2F2, MDM2, MDM4, and MYC.*

This measure has the advantage that such cellular senescence-inhibiting cell cycle regulator genes are analyzed which, according to the findings of the inventors, have particular predictive power.

*"CCND1"* encodes the so-called cyclin D1 protein. Cyclin D1 belongs to the highly conserved cyclin family, whose members are characterized by a periodicity in protein abundance throughout the cell cycle. Cyclin D1 forms a complex with and functions as a regulatory subunit of CDK4 or CDK6, whose activity is required for cell cycle G1/S transition. This protein has been shown to interact with tumor suppressor protein Rb and the expression of this gene is regulated positively by Rb. Mutations, amplification and overexpression of this gene are observed frequently in a variety of tumors and may contribute to tumor genesis.

"CCND2" encodes the cyclin D2 protein. Cyclin D2 also belongs to the cyclin family. It forms a complex with and functions as a regulatory subunit of CDK4 or CDK6, whose activity is required for cell cycle G1/S transition. This protein has been shown to interact with and be involved in the phosphorylation of tumor suppressor protein Rb. High level expression of this gene was observed in ovarian and testicular tumors.

The "CCND3" gene encodes the cyclin D3 protein. This cyclin forms a complex with and functions as a regulatory subunit of CDK4 or CDK6, whose activity is required for cell cycle G1/S transition. Cyclin D3 has been shown to interact with and be involved in the phosphorylation of tumor suppressor protein Rb. The CDK4 activity associated with this cyclin was reported to be necessary for cell cycle progression through G2 phase into mitosis after UV radiation. Mutations in *CCND3* are implicated in cases of breast cancer.

"CDK4" encodes the cyclin-dependent kinase 4 also known as cell division protein kinase 4. It is a member of the cyclin-dependent kinase family. It is a catalytic subunit of the protein kinase complex that is important for cell cycle G1 phase progression. The activity of this kinase is restricted to the G1-S phase, which is controlled by the regulatory subunits D-type cyclins and CDK inhibitor p16^{INK4a}. This kinase was shown to be responsible for the phosphorylation of Rb. Cyclin D-CDK4 complexes are major integrators of various mitogenic and antimitogenic signals. Mutations in this gene as well as in its related proteins including D-type cyclins, p16^{1NK4a} and Rb were all found to be associated with tumorigenesis of a variety of cancers.

The "CDK6" gene encodes cyclin-dependent kinase 6, another member of the cyclin-dependent kinases. This kinase is a catalytic subunit of the protein kinase complex, important for the G1 phase progression and G1/S transition of the cell cycle and the complex is composed also by the activating sub-unit cyclin D. The activity of this kinase first appears in mid-G1 phase, which is controlled by the regulatory subunits including D-type cyclins and members of INK4 family of CDK inhibitors. This kinase, as well as CDK4, has been shown to phosphorylate, and thus regulate the activity of tumor suppressor Rb making CDK6 an important protein in cancer development.

*"CCNE1"* encodes cyclin E1 protein, which is a member of the conserved cyclin family. This cyclin forms a complex with and functions as a regulatory subunit of CDK2, whose activity is required for cell cycle G1/S transition. This protein accumulates at the G1-S phase boundary and is degraded as cells progress through S phase. Overexpression of this gene has been observed in many tumors, which results in chromosome instability, and thus may contribute to tumor genesis.

The "E2F2" gene encodes the protein E2F2. E2F2 is a member of the E2F family of transcription factors. The E2F family plays a crucial role in the control of cell cycle and action of tumor suppressor proteins and is also a target of the transforming proteins of small DNA tumor viruses. E2F2 is activated by p27 and phosphorylated by Rb1.

"*MDM2*" encodes the 'mouse double minute 2' homolog. It is an important negative regulator of the p53 tumor suppressor. The gene has been identified as an oncogene. Several human tumor types have been shown to have increased levels of MDM2 protein, including soft tissue sarcomas and osteosarcomas as well as breast tumors. The MDM2 oncoprotein ubiquitinates and antagonizes p53. Suppression of MDM2 is therefore crucial for the activation of p21 and the suppression of CDK4/6.

The "*MDM4*" gene encodes the 'mouse double minute 4' homolog. MDM4 protein has been shown to interact with E2F1, MDM2 and p53. Suppression of MDM4 is crucial for the activation of p21 and the suppression of CDK4/6.

The "*MYC*" gene, also referred to as c-myc, is a member of the family of regulator genes and proto-oncogenes that code for transcription factors. *MYC* was the first gene to be discovered in this family. In cancer *MYC* is often persistently expressed. This leads to the increased expression of many genes, some of which are involved in cell proliferation, contributing to the formation of cancer.

Again, the human variants of the above-referenced genes and gene products are preferred.

In an embodiment of the invention in step (3) said classification as a non-responder occurs if a genetic modification is identified as present in at least 1, or at least 2, or at least 3, at least 4, or at least 5, or at least 6, or at least 7, or at least 8, or at least 9, or at least 10, or at least 11, or at least 12 different cellular senescence-inducing cell cycle regulator genes and/or senescence-inhibiting cell cycle regulator genes.

This measure has the advantage that by increasing the number of genes the diagnostic or classifying certainty is increasingly enhanced. However, it turned out that already a small number of genes might be sufficient, e.g. 1, 2, or 3, 4 of each group, for a reliable diagnosis or classification which may result in therapeutic consequences. Especially if the genetic modification is present in any of the genes CDK4, CDK6, MDM2 or MDM4 the diagnostic or classifying certainty is very high even if the modification is present in only one or two or three of these genes.

In embodiments of the invention said patient is suffering from a melanoma, preferably a metastatic melanoma, and/or from a carcinoma, preferably a metastatic carcinoma, and/or from a lymphoma.

This measure has the advantage that the method according to the invention is adapted to the classification of such a patient where the identified genes are of particular prognostic significance.

Another subject-matter of the invention relates to the use of a gene set comprising (i) cellular senescence-inducing cell cycle regulator genes, and (ii) cellular senescence-inhibiting cell cycle regulator genes for classifying a patient in need as non-responder or responder to immune checkpoint inhibitor therapy.

The embodiments, features, limitations, and advantages mentioned for the method according to the invention likewise apply to the use according to the invention.

It is to be understood that the before-mentioned features and those to be mentioned in the following cannot only be used in the combination indicated in the respective case, but also in other combinations or in an isolated manner without departing from the scope of the invention.

The invention includes the following aspects defined in the following clauses which form part of the present description, which, however, do not represent claims, in accordance with the decision J15/88 of the Legal Board of Appeal of the European Patent Office.
1. A method ex vivo for classifying a patient in need as non-responder or responder to immune checkpoint inhibitor therapy, comprising the following steps:
   1) providing a biological sample originating from said patient;
   2) analyzing the biological sample for the presence or absence of a genetic alteration causing a modification of function in
      - cellular senescence-inducing cell cycle regulator genes, and/or
      - cellular senescence-inhibiting cell cycle regulator genes, 3) classifying the patient as
      - non-responder if in step (2) said genetic alterations are identified as present, or
      - responder if in step (2) said genetic alterations are identified as absent.
2. The method of clause 2, wherein said genetic alteration causing a modification of function is a
   - loss-of-function mutation in cellular senescence-inducing cell cycle regulator genes, and/or
   - gain-of-function mutation in cellular senescence-inhibiting cell cycle regulator genes.
3. The method of clause 1 or 2, wherein said cellular senescence-inducing cell cycle regulator genes are selected from the group consisting of: CDKN2A, CDKN2B, CDKN2C, CDKN1A, CDKN1B, RB1, TP53, STAT1, JAK1, JAK2, and JAK3.
4. The method of any of the preceding clauses, wherein said cellular senescence-inhibiting cell cycle regulator genes are selected from the group consisting of: CCND1, CCND2, CCND3, CDK4, CDKN1B, CDK6, CCNE1, RB1, E2F2, MDM2, MDM4, and MYC.
5. The method of any of the preceding clauses, wherein in step (3) said classification as a non-responder occurs if a genetic modification is identified as present in at least 1, or at least 2, or at least 3, or at least 4, or at least 5, or at least 6, or at least 7, or at least 8, or at least 9, or at least 10, or at least 11 or at least 12 different cellular senescence-inducing cell cycle regulator genes and/or senescence-inhibiting cell cycle regulator genes.
6. The method of any of the preceding clauses, wherein said patient is suffering from a melanoma, preferably a metastatic melanoma.
7. The method of any of the preceding clauses, wherein said patient is suffering from a carcinoma, preferably a metastatic carcinoma.
8. The method of any of the preceding clauses, wherein said patient is suffering from a lymphoma.
9. Use of a gene set comprising (i) cellular senescence-inducing cell cycle regulator genes, and (ii) cellular senescence-inhibiting cell cycle regulator genes for classifying a patient in need as non-responder or responder to immune checkpoint inhibitor therapy.
10. Use of clause 9, wherein said cellular senescence-inducing cell cycle regulator genes are selected from the group consisting of: CDKN2A, CDKN2B, CDKN2C, CDKN1A, CDKN1B, RB1, TP53, STAT1, JAK1, JAK2, and JAK3.
11. Use of clause 9 or 10, wherein said cellular senescence-inhibiting cell cycle regulator genes are selected from the group consisting of: CCND1, CCND2, CCND3, CDK4, CDK6, CCNE1, E2F2, MDM2, MDM4, and MYC.
12. Use of any of clauses 9-11, wherein said gene set comprises at least 1, preferably at least 2, further preferably at least 3, further preferably at least 4, further preferably at least 5, further preferably at least 6, further preferably at least 7, further preferably at least 8, further preferably at least 9, further preferably at least 10, highly preferably at least11 different cellular senescence-inducing cell cycle regulator genes and/or senescence-inhibiting cell cycle regulator genes.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner in the context of any embodiment of the invention.

The invention is now described and explained in further detail by referring to the following non-limiting examples and figures.

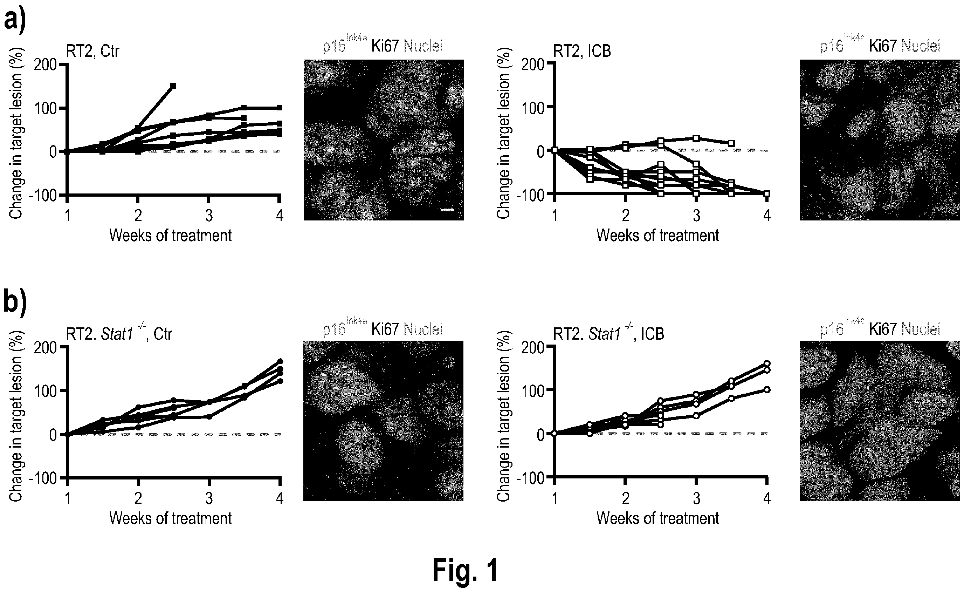

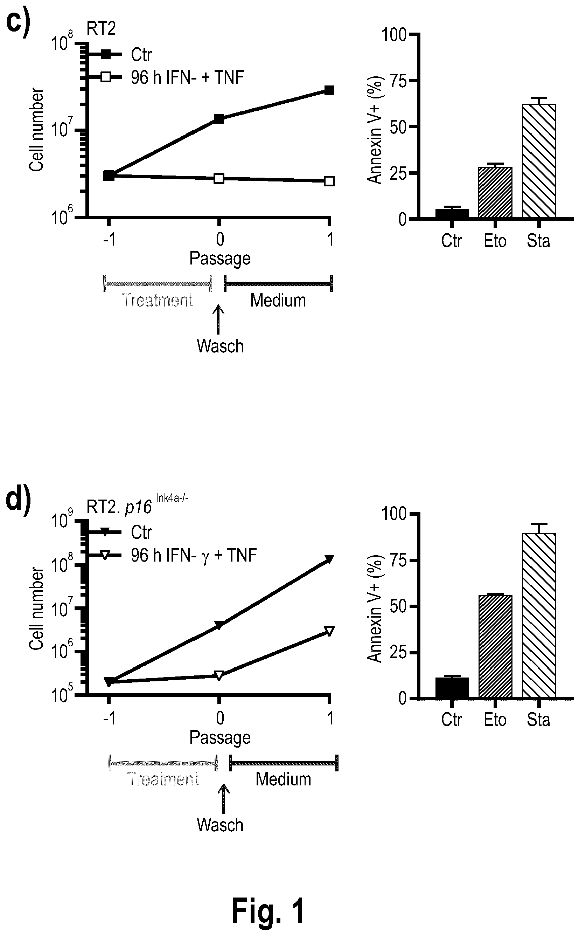

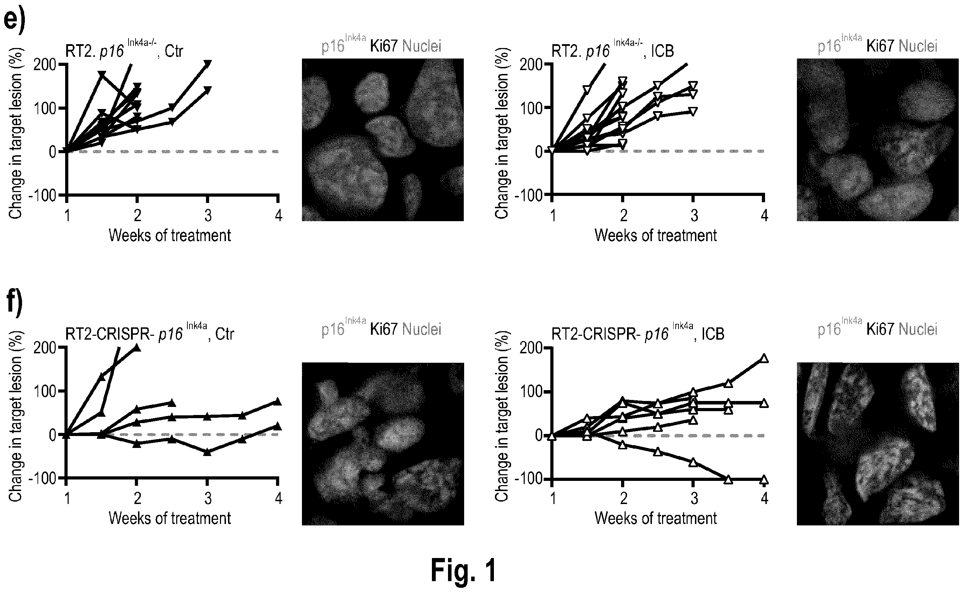

- Fig. 1: *p16^{lnk4a}*-dependent immune control of transplanted RT2 cancers. a, b, e, f, Cancer cells derived from RT2 mice (a), RT2.*Stat1*^{-/-} (b) mice, or RT2.p16^{lnk4a-/-} cancer cells (e), or RT2-CRISPR-p16^{lnk4a-/-} cancer cells (e) were transferred s.c. into syngeneic mice. Treatment was started when tumours where > 3 mm in diameter. Cancer growth was measured in the absence (Ctr) or presence of immune checkpoint inhibitors (ICB). The spider blot shows the diameter change of the target lesion of each metastasis. Representative images (from N=3 tumours) show p16^{lnk4a} (red), Ki67 (blue) and nuclei (green). Scale bar 2 µm. c, d, For the senescence assay RT2-cancer cells (c) or RT2.p16^{lnk4a-/-}cancer cells (d) were cultivated with medium (Ctr) or medium containing 100 ng ml-1 IFN-γ and 10 ng ml-1 TNF for 96 h, washed and then cultivated with medium for another 4 days, data are the logarithmic mean of vital cells from four measurements (c, d, left). Cells were exposed to either medium (Ctr) or etoposide (Eto, 100 µM) or staurosporine (Sta, 0.5 µM) for 24 h for apoptosis induction and stained for Annexin V. Positive cells were detected by flow cytometry; data show the geometric mean of three replicates with geometric SD (c, d, right).

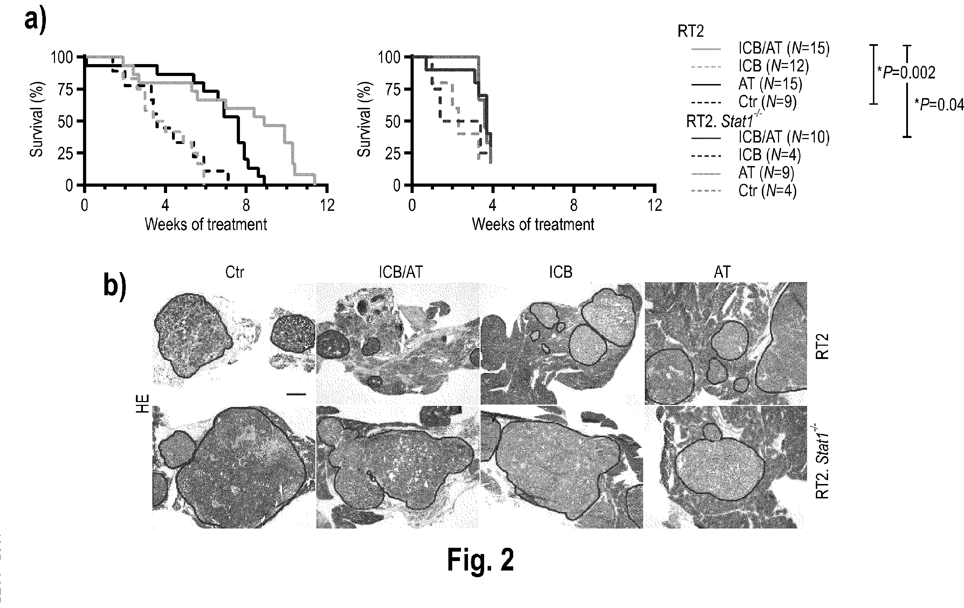

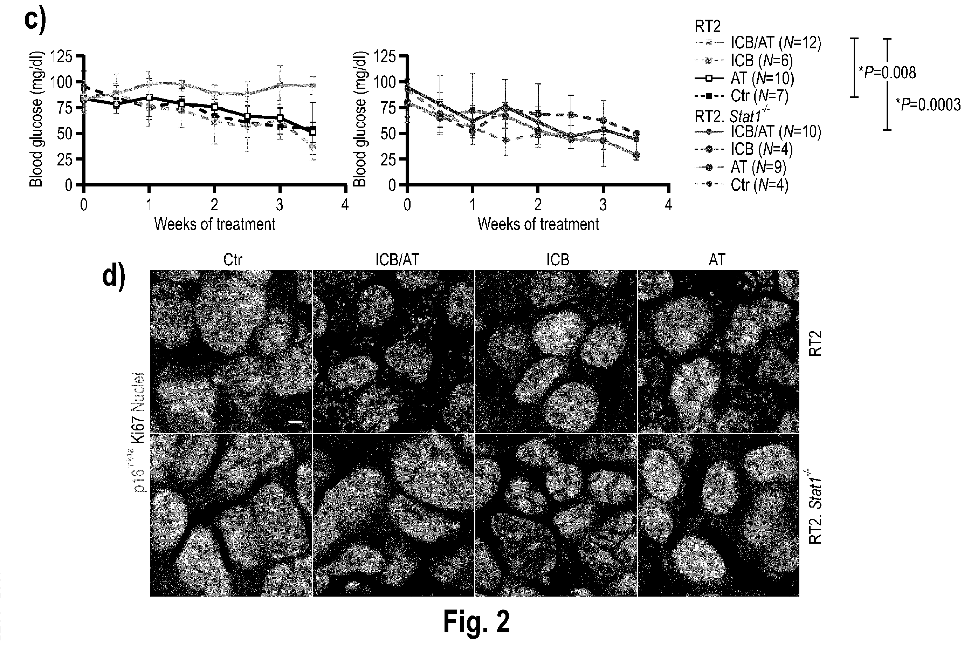

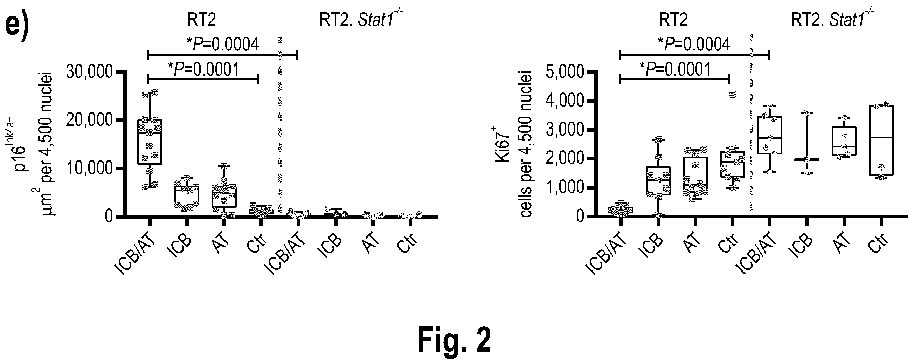

- Fig. 2: *Stat1*-dependent immune control of advanced endogenous RT2 cancers and induction of senescence by cytotoxic immune responses. a, Survival curves of RT2- or RT2.*Stat1*^{-/-} mice treated with either isotype mAbs (Ctr), with Tantigen-specific CD4⁺ T_{H}1 cells (AT), with immune check-point inhibitors (ICB) or ICB- and AT (ICB/AT). b, Cancer size shown by representative HE stainings of pancreas of RT2 (upper panel) or RT2.*Stat*^{*1-*/*-*} mice (lower panel) after four weeks of treatment. Mice were treated as described in (a). Green lines depict the size of RT2-cancers after treatment. Ar week 4 ICB/AT-treated mice hat already a two-fold reduction in the islet sice. c, Time course of blood glucose levels of RT2-or RT2.*Stat*^{*1-*/*-*} mice (median ± interquartile range) treated as described in (a). The mice were sacrificed after 4 weeks of treatment for ex *vivo* analysis. The area under the curve was calculated for statistical comparison using Fisher's exact test. d, e, Triple-staining for the senescence marker p16^{lnk4a} (red) and the proliferation marker Ki67 (blue) and for nuclei (green) (d), and box plots with individual data points (e) showing quantification of p16^{lnk4a} (left) or Ki67⁺ (right) cancer cells of individual mice treated as described in (a). Each point represents triplicates from one mouse. Significance tested by using Log Rank test (a, left) Fishers exact test (a, right, RT2.*Stat*^{*1-*/*-*} mice have been censored after 3.7 weeks of treatment), two-tailed Mann-Whitney test (c, e). Scale bars 200 pm (b) or 2 pm (d).

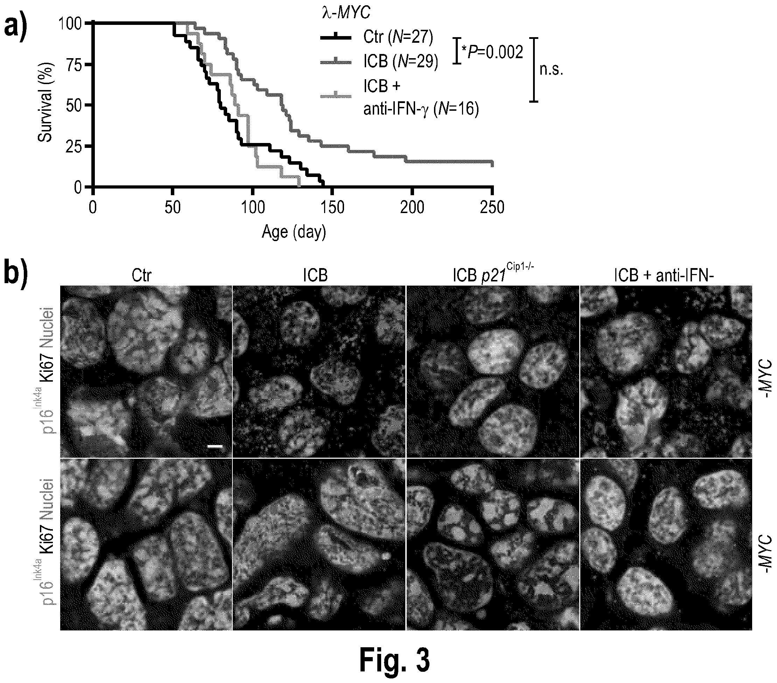

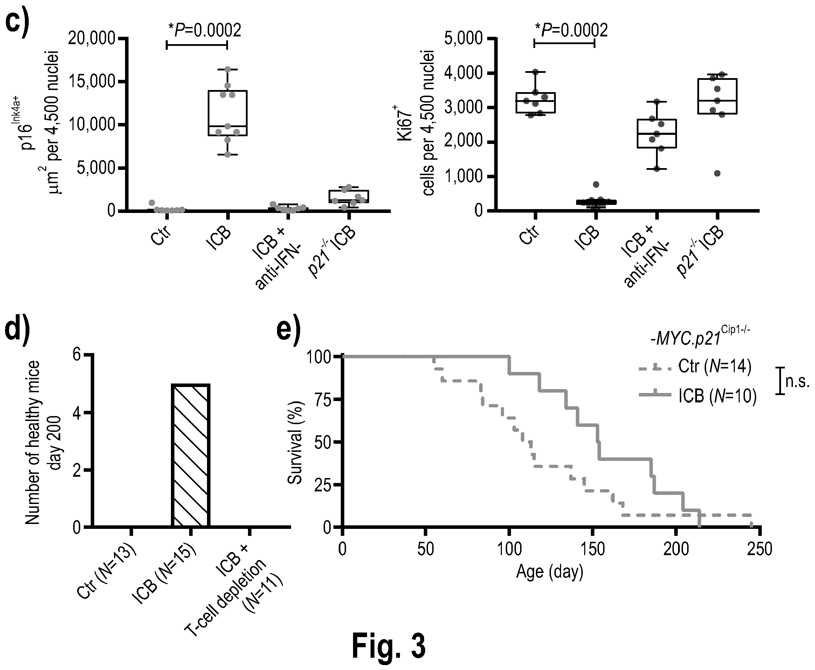

- Fig. 3: *p21^{CiP1}*-dependent immune control of λ-*MYC-*induced lymphomas. a, Survival curves of untreated (Ctr), or immune check-point inhibitor (ICB)-treated *λ-MVC* mice, either in the absence (ICB) or presence of anti-IFN-γ mAb (ICB + anti-IFN-γ). b, c, Triple-staining for the senescence marker p16^{lnk4a} (red, upper panel) or p21^{Cip1} (red, lower panel), the proliferation marker Ki67 (blue), and for nuclei (green); scale bar 2 pm; pictures are representative examples (b). Box plots with individual data points representing p16^{lnk4a+} (c, left) or Ki67+ nuclei (c, right) of B cells from Ctr-, ICB-, ICB- and anti-IFN-γ-treated *λ-MVC* mice, or ICB-treated λ-*MYC.p21*^{*Cip1-*/*-*} mice. Lymph nodes were isolated at similar ages. Each point represents triplicates from one mouse. d, Number of healthy mice at day 200, all mice remained healthy beyond day 250. e, Survival curves of either untreated or ICB-treated λ*-MYC.p21*^{*Cip1-*/*-*} mice. Significance tested by using Log Rank test (a, d), two-tailed Mann-Whitney test (c).

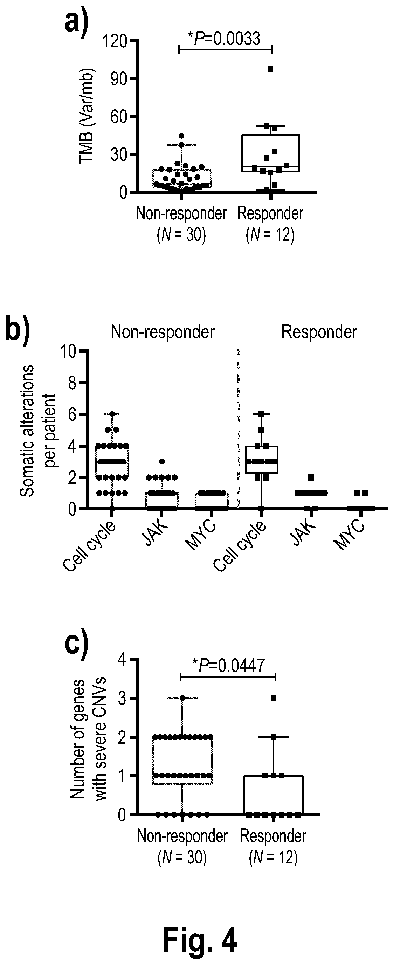

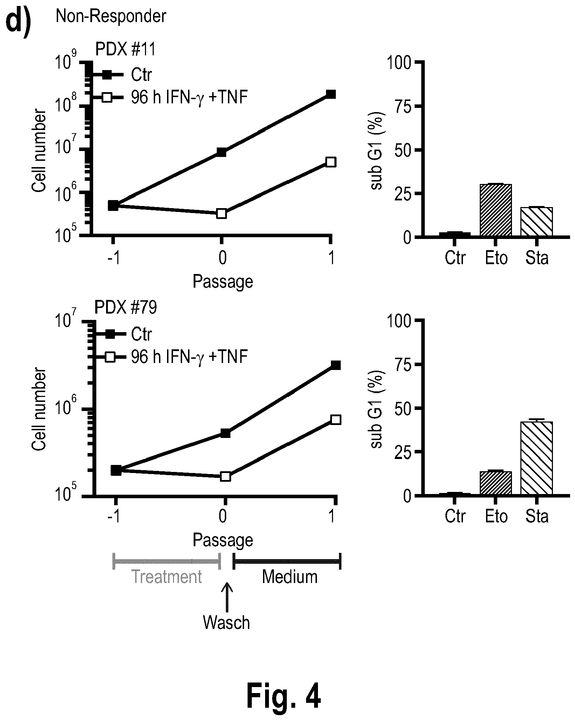

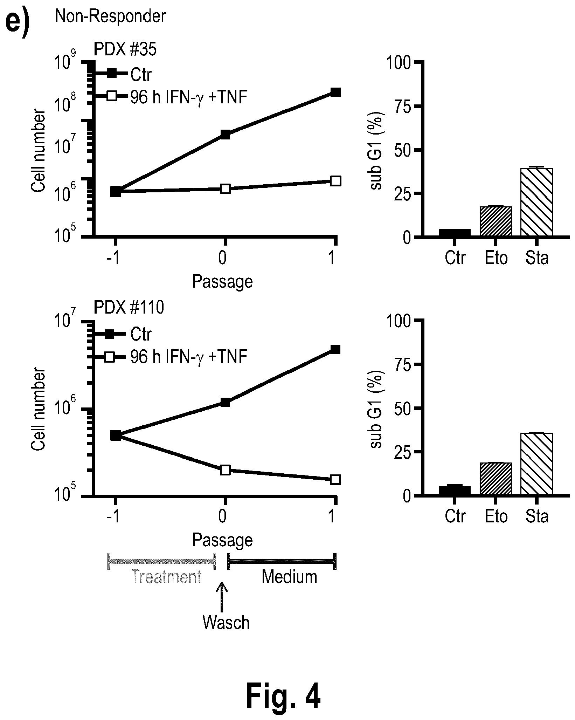

- Fig. 4: Loss of senescence-inducing cell cycle regulator genes selectively in metastatic melanomas of patients not responding to cancer immunotherapy. a, b, c, Sequencing data from 30 non-responder patients versus 12 responder patients. Non-responders were patients where disease progressed within 3 months of ICB therapy. Responders were patients where metastases regressed > 1 year of ICB therapy. a, Tumour mutational burden (TMB, copy number variations). b, Number of tumour-specific alterations in 19 genes of the cell cycle, Jak or Myc pathway. c, Number of genes with homozygous deletions in cycle inhibitors or amplifications 2 3 in cell cycle promoters. Genes analysed: *CDKN2A*/*B*/*C, CDKN1A*/*B, RB1, TP53, JAK1*/*2*/*3, CCND1*/*2*/*3, CDK4*/*6, CCNE1, MDM2*/*4, MYC.* d, e, left, Patient derived cell lines were cultivated for the senescence assay with medium (Ctr) or with medium containing 100 ng ml⁻¹ IFN-γ and 10 ng ml⁻¹ TNF for 96 h, washed and then cultivated with medium for another 4 - 6 days. Data are the logarithmic mean of vital cells from four measurements. d, e, right, Cells were exposed to either medium (Ctr) or etoposide (Eto, 100 µM) or staurosporine (Sta, 0.5 µM) for 24 h for apoptosis induction and stained with propidium iodide. SubG1 cells were detected by flow cytometry analysis; data show the geometric mean of three replicates with geometric SDS. Significance tested by using two-tailed Mann-Whitney test (a-c).

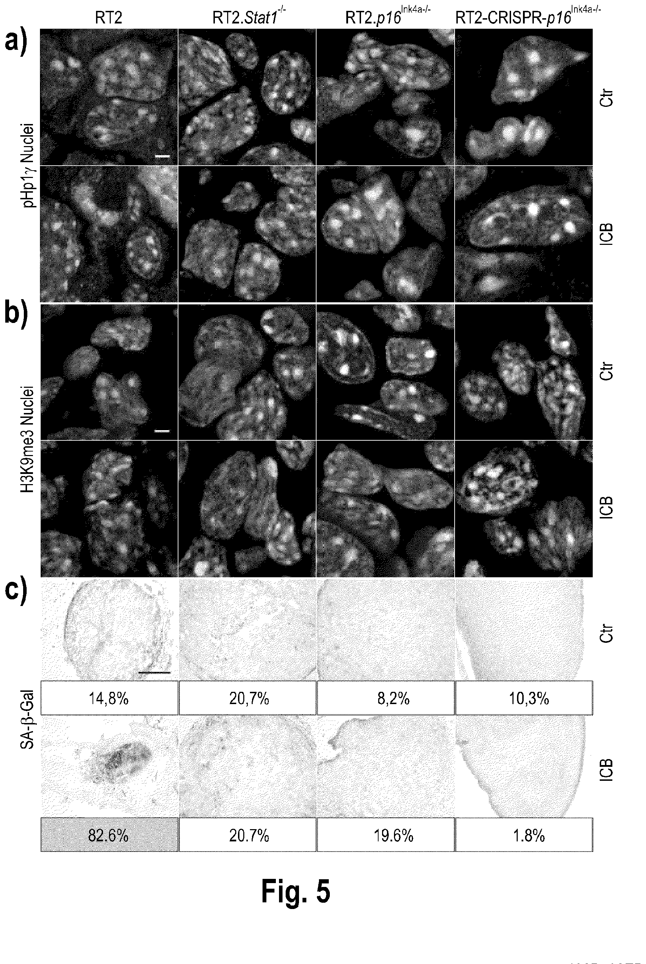

- Fig. 5: Immune control of transplanted cancers (Ctr) induces the expression of the senescence markers pHP1y, H3K9me3 or SA-β-gal in RT2 but not in *Stat1-* and p16-deficient cancers. Immunofluorescence microscopy images of nuclei of transplanted pancreatic islet cancers from either RT2, RT.*Stat1*^{*-*/*-*} or RT2.*p16*^{*lnk*4a-/-} cancer cells. Mice were treated with isotype control mAbs (Ctr) or with immune checkpoint blockade (ICB). a pHP1γ (red), nuclei (white). b, H3K9me3 (red), nuclei (white). c, SA-β-gal activity at pH 5.5 and percentages of SA-β-gal positive tumor cells embedded in corresponding color dield (panel below). Scale bars 2 µm (a,b), 1000 µm (c).

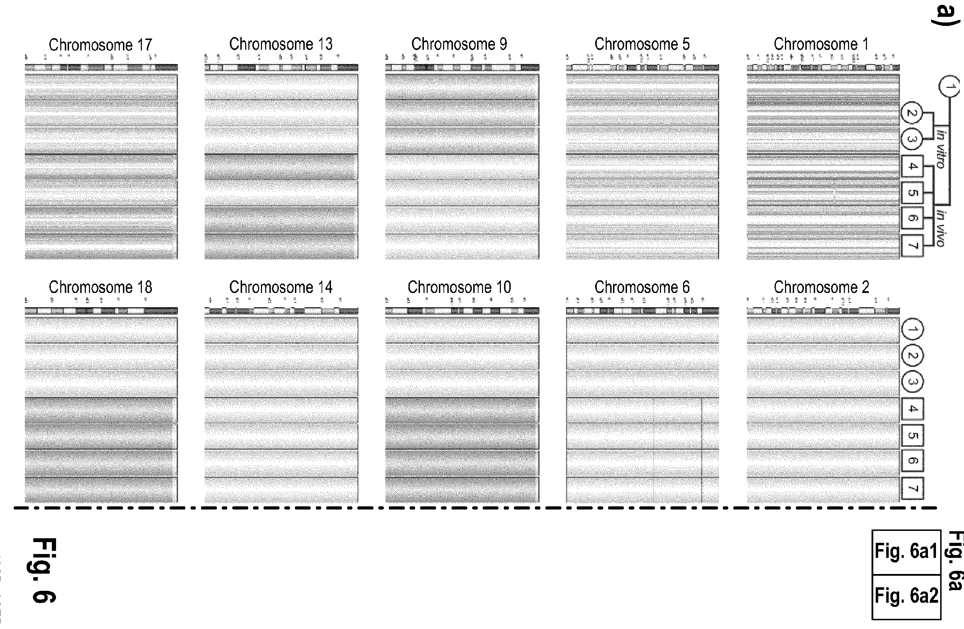

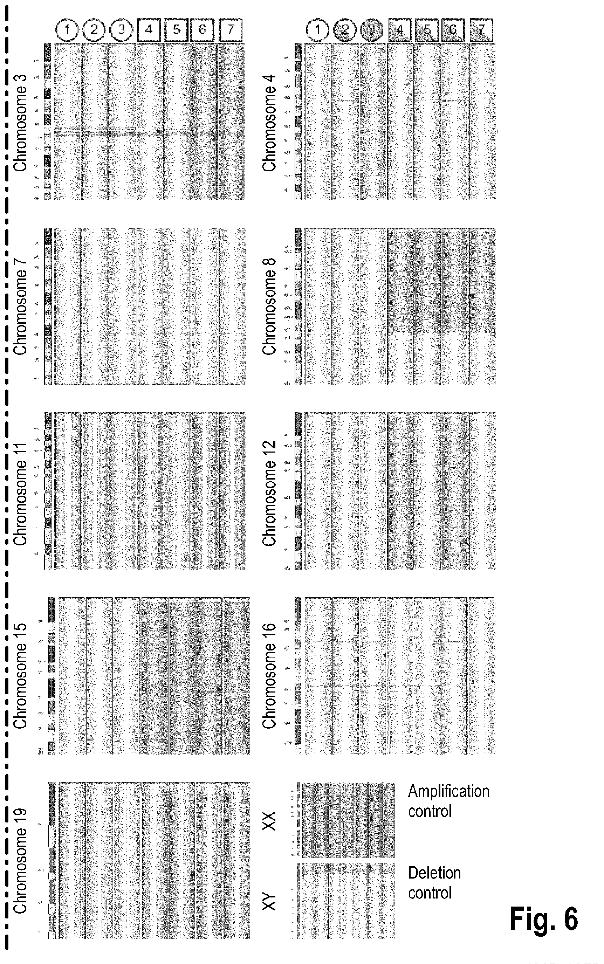

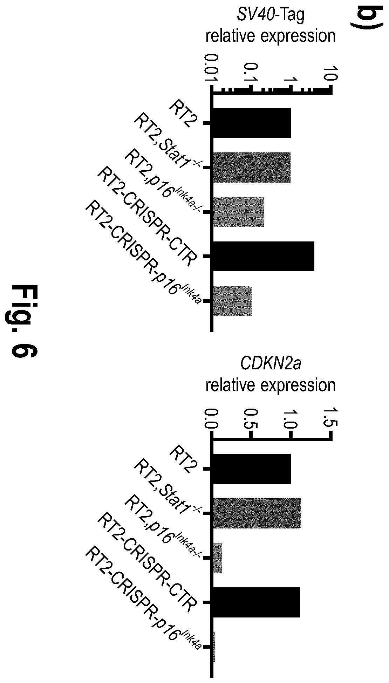

- Fig. 6: *CDKN2a* loss variants of RT2 cancer cells. a, Comparative genomic hybridization arrays of 6 variant RT2 cell lines (2-7) and the primary RT2 cell line (1) compared with the chromosome ideogram of wildtype C3HeB/FeJ mice. Amplifications in red, deletions in green, genome of interest from female mice (XX) compared to reference DNA of male mice (XY). The *CDKN2a*-loss variants on chromosome 4, qC4.A were generated by selection of cells resistant to immunotherapy (2 and 3 *in vitro*, 4-*7 in vivo*). b, Relative *SV40*-Tag (left) and *CDKN2a* (right) expression in RIP-Tag2 (set as 1), RIP-Tag2.Stat*1*^{-/-}, RT2.*p16*^{*lnk4a-*/-}, RT2-CRISPR-Ctr or RT2-CRISPR-*p16lnk*4a cells as determined by qRT-PCR.

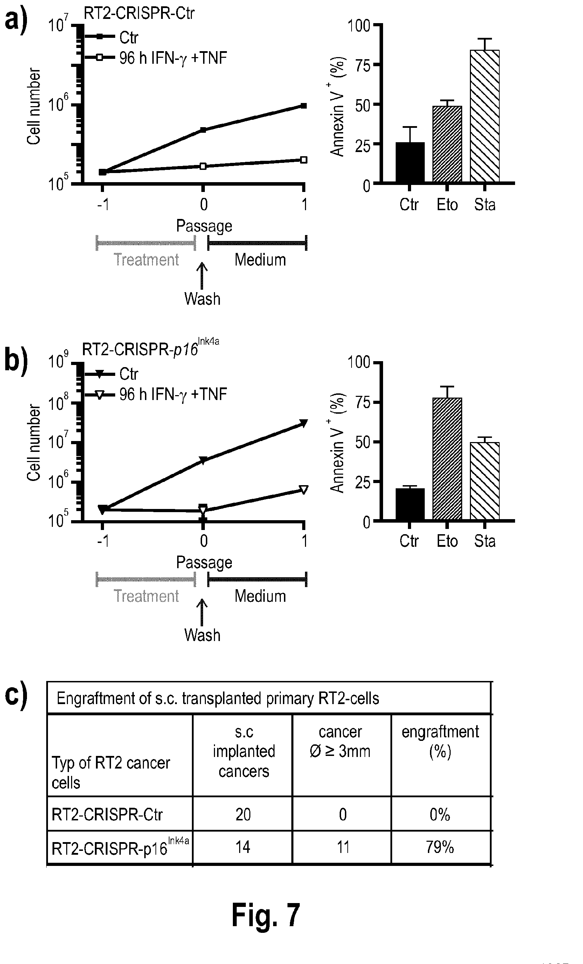

- Fig. 7: Similar *in vitro* growth dynamics and apoptosis sensitivity but different resistance to CIS of RT2-cancer cells that were transfected with either an empty CRISPR-Cas vector or CRISPR-Cas-p16^{lnk4a}, respectively. a, b, RT2-CRISPR-Ctr cancer cells (a) or RT2-CRISPR-p16^{lnk4a} cancer cells (b) were cultivated for the senescence assay with medium (Ctr) or with medium containing 100 ng ml⁻¹ IFN-γ and 10 ng ml⁻¹ TNF for 96 h, washed and then cultivated with medium for another 4 days; data are the logarithmic mean of vital cells from four measurements (a, b, left). Cells were exposed to either medium (Ctr) or etoposide (Etc, 100 pM) or staurosporine (Sta, 0.5 pM) for 24 h for apoptosis induction and stained for Annexin V. Positive cells were detected by flow cytometry analysis; data show the geometric mean of three replicates with geometric SD (a, b, right). c, Number of mice with so transplanted RT2-cancer cells (1 × 10⁶) and engraftment efficacy of either RT2-CRISPR-Ctr- or RT2-CRISPR-p16*lnk4a* cancer cells.

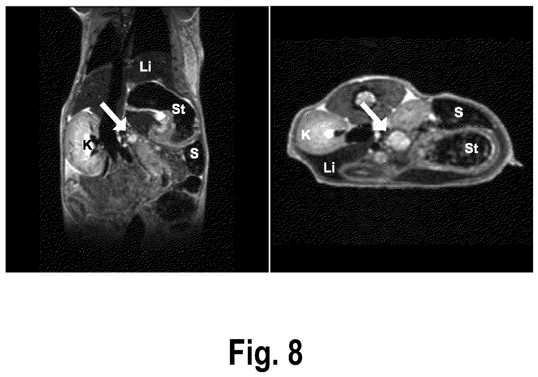

- Fig. 8: Advanced pancreatic islet cancers in RIP-TagZ mice (RT2-cancers) at 10 weeks of age, at the beginning of ICB-therapy. Representative magnetic resonance images of a RIP-Tag2 mouse at 10 weeks of age. Coronal view (left), transversal view (right). Arrows point towards RT2-cancers. Spleen (S), stomach (St), kidney (K), liver (Li).

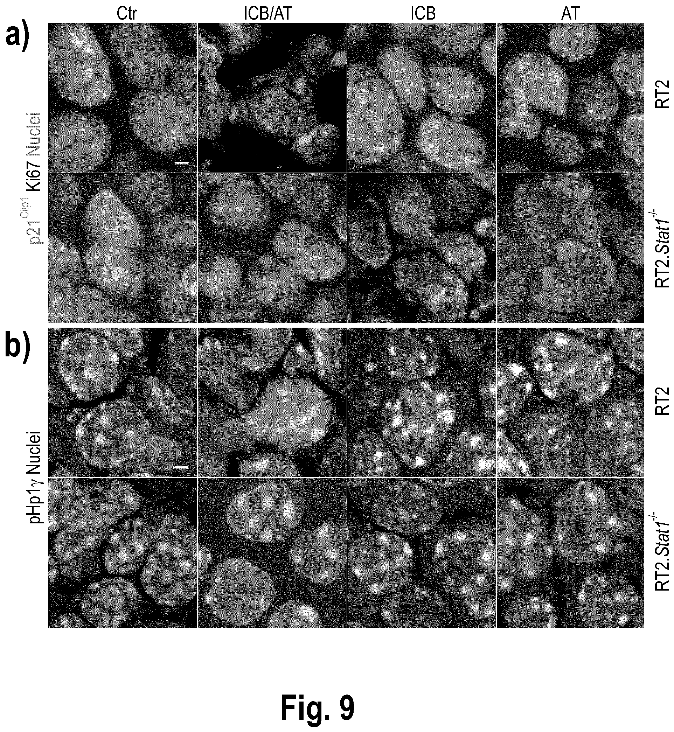

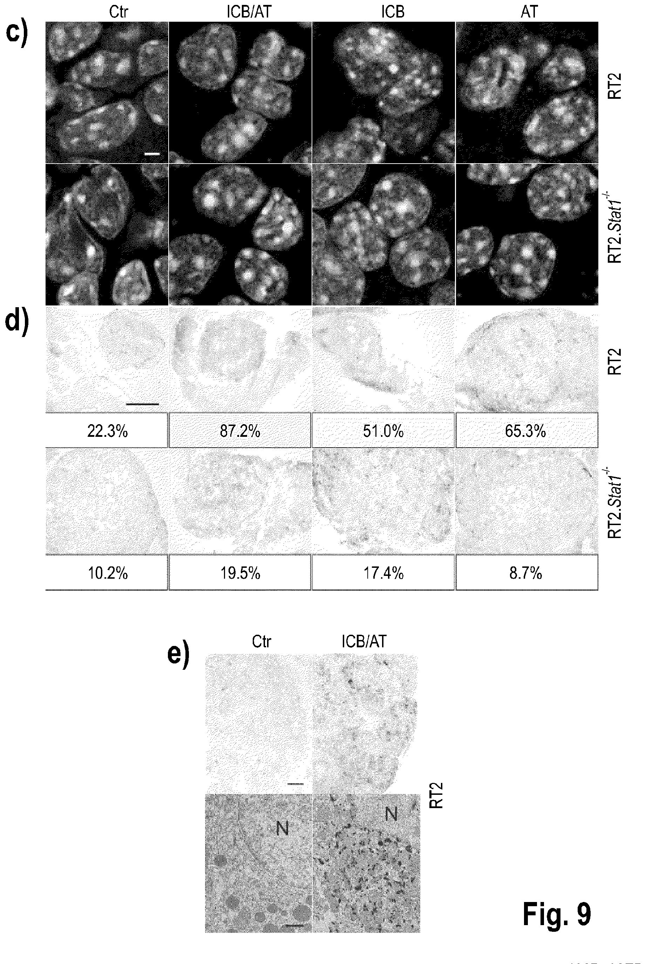

- Fig. 9: *Stat1*-dependent induction of p21^{Cip1} and other senescence marker in RT2-cancers by strong cytotoxic immune responses. Representative fresh frozen cryostat sections of RT2 cancers from either RT2 or RT2.*Stat1*^{-/-} mice with established cancers where treated with isotype control (Ctr) or immune checkpoint blockade and adoptive T-cell transfer (ICB/AT) or with either ICB or AT. a, p21^{Cip1} (red), Ki67 (blue), nuclei (green). b, pHP1γ (red), nuclei (white). c, H3K9me3 (red), nuclei (white). d, SA-β-gal activity at pH5.5 and percentages of SA-β-gal positive tumor cells embedded in corresponding color field (panel below). e, Electron microscopy (EM) of SA-β-gal-stained RT2-cancers. Scale bars 2 pm (a-c), 1000 um (d), 1 pm (e).

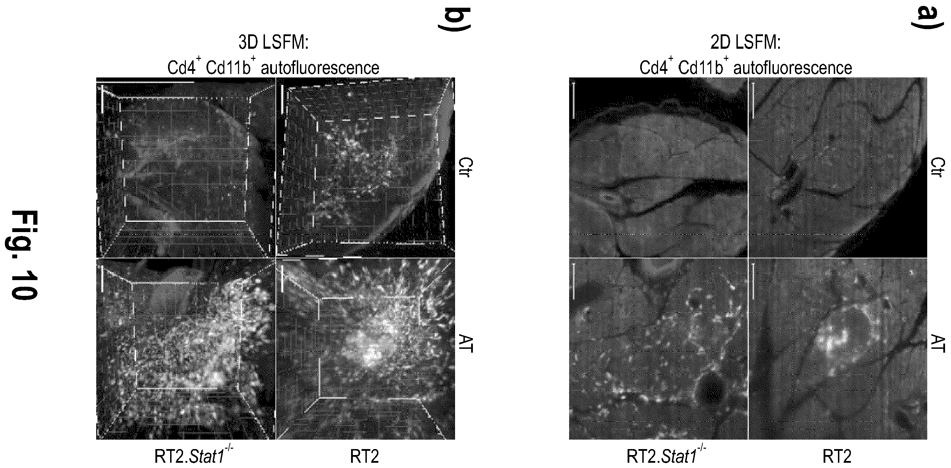

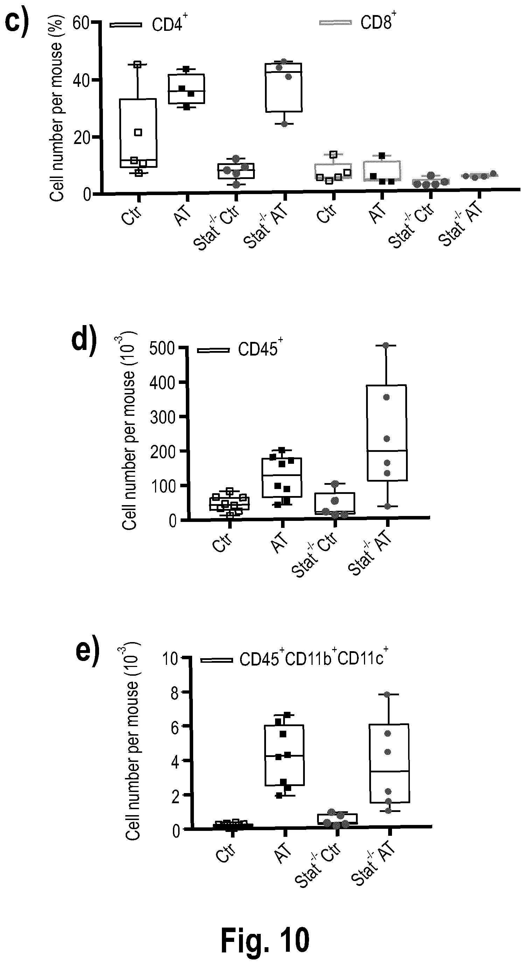

- Fig. 10: Tumor microenvironment of either RT2 or RT2.*Stat1*^{-/-} mice following AT. Pancreas from either RT2 or RT2.*Stat1*^{-/-} mice were isolated 2 days after the second treatment with isotype control (Ctr) or adoptive T-cell transfer (AT), a, In vivo 2D light sheet fluorescence microscopy images (LSFM) b, 3D LSFM, CD4⁺ (red), CD11b⁺ (green), autofluorescence (blue). Labelled mAbs were injected 2 h prior to harvesting organs, scale bars 100 um. c-e, Flow cytometry analysis of tumor-infiltrating leucocytes of RT2 or RT2.*Stat1*^{-/-} pancreas 2 days after the second adoptive T cell transfer (AT); c, percentage of CD4⁺ and CD8⁺ T cells; Cl, number of CD45⁺ immune cells; e, number of CD45⁺CD11b⁺CD11c⁺ dendritic cells. Each point represents one mouse.

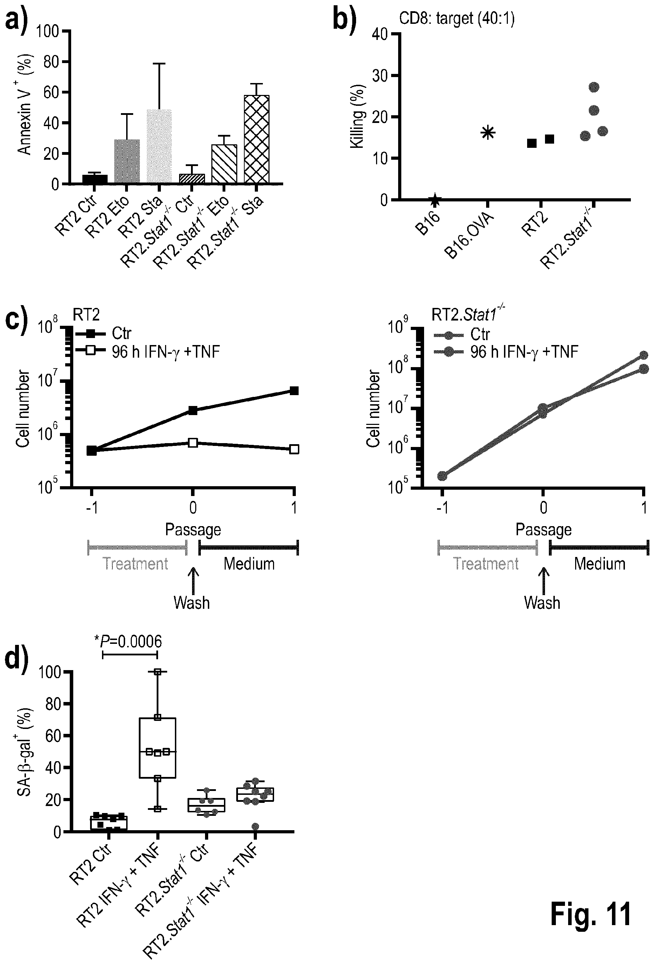

- Fig. 11: *Stat1* is required for senescence induction but neither for apoptosis nor T cell-mediated killing of RT2-cancer cells. a, RT2 (N=4) or RT2.*Stat1*^{*-*/*-*}cancer cells (*N*=4) were exposed to either medium (Ctr), etoposide (Eto, 100 pM), or staurosporine (Sta, 0.5 pM) for 24 h for apoptosis induction and stained for Annexin V. Positive cells were detected by flow cytometry analyses; data show the geometric mean of three replicates with geometric SD (0, d, right). b, B16-F1O or OVA-expressing B16 melanoma cells (C57BL/6), or RT2 or *Stat1*^{*-*/*-*} RT2-cancer cells (C3HeB/FeJ) were cultivated with OVA-reactive C57BL/6 CD8⁺ cytotoxic T cells and specific chromium release was determined after 1.5 h. c, Senescence assay: RT2 (left) or RT2.*Stat1*^{-/-} (right) cancer cells were cultivated with medium (Ctr) or with medium containing 100 ng ml⁻¹ IFN-γ and 10 ng ml⁻¹ TNF for 96 h, washed and then cultivated with medium for another 4 days; data are the logarithmic mean of vital cells from four measurements. d, Staining for SA-β-gal. Significance tested by using two-tailed Mann-Whitney test.

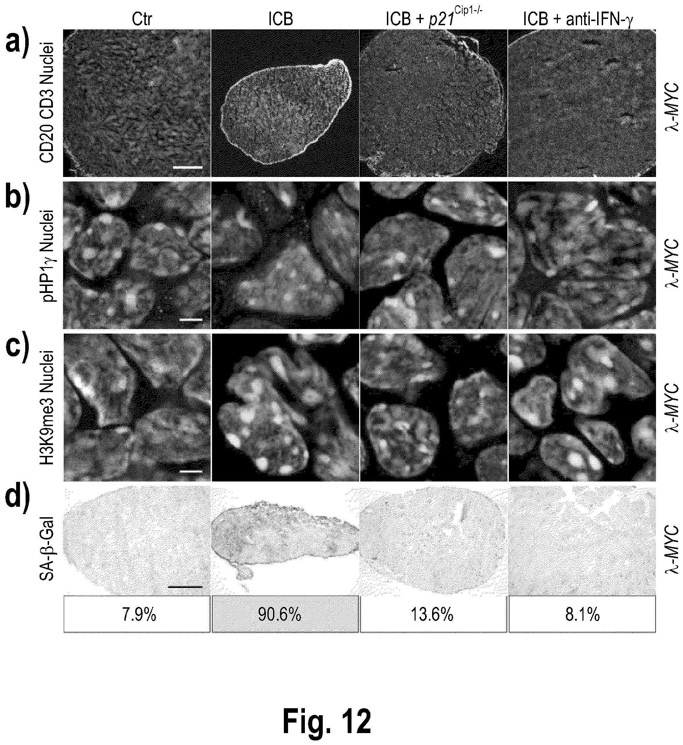

- Fig. 12: Immune control of *λ-MVC* mice preserves the normal lymph node structure and induces a senescent B-cell phenotype in a p21^{Cip1}-dependent manner. Fresh frozen cryostat sections of representative lymph nodes of λ-*MYC* or λ*-MYC.p21*^{*CiP1-*/*-*} mice. *λ-MVC* mice were untreated (Ctr) or treated with anti-CTLA-4 and anti-PD-1 mAbs (ICB) or anti-CTLA-4, anti-PD-1 and anti-IFN-γ mAbs (ICB + anti-IFN-γ). λ*-MYC.p21*^{*CiP1-*/*-*} mice were ICB treated (ICB *p21*^{*Cip1-*/*-*}). a, CD20 (red), CD3 (green), nuclei (white); scale bar, 500 µm. b, pHP1γ (red), nuclei (white). c, H3K9me3 (red), nuclei (white). d, SA-β-gal activity at pH5.5 and percentages of SA-β-gal positive tumor cells embedded in corresponding color field (panel below).

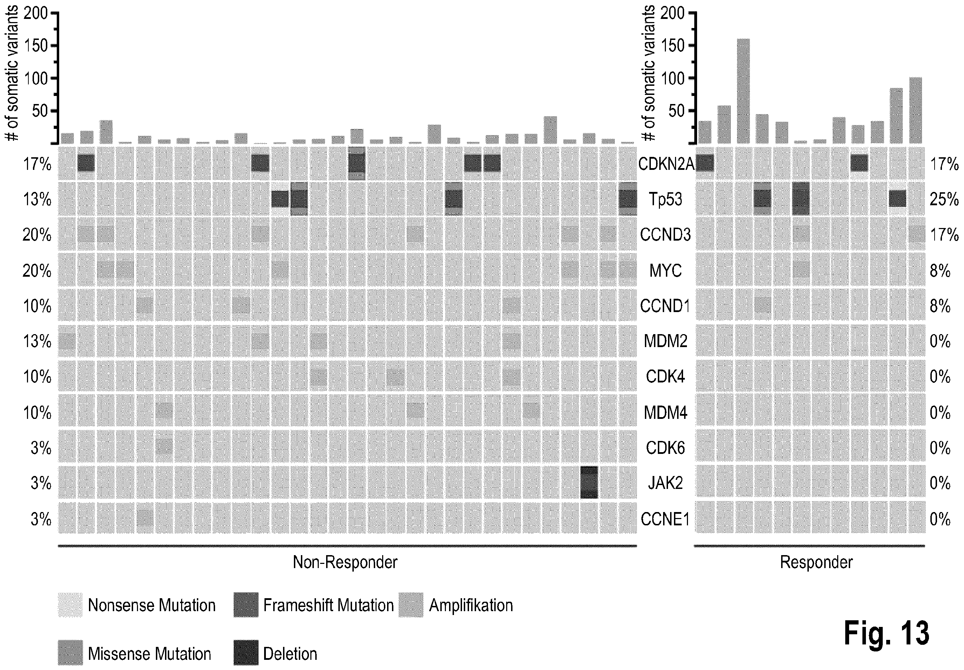

- Fig. 13: Mutation Oncoplot with loss of function and gain of function alterations in the genes of the cell cycle control. Genes with severe somatic alterations in the genes of the cell cycle control. The mutation frequency for each gene in each group is either on the left (non-responder) or right (responder) site of the oncoplot. No changes in CCND2, CDKNZB, CDKNZC, CDKN1a, CDKN1 B, RB1, JAK1 or JAK3 were detected.

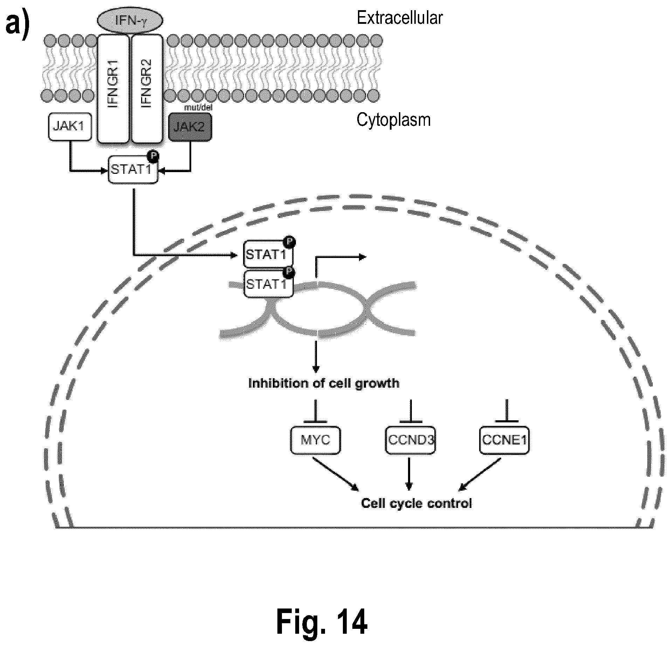

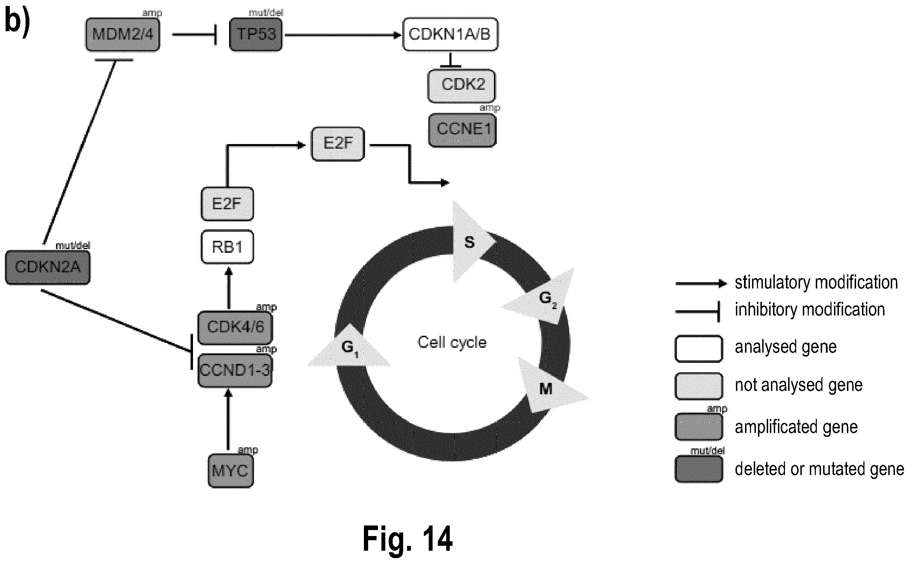

- Fig. 14: IFN-JAK1-STAT1 signaling pathway leads to cell cycle control. a, IFN-receptor signalling. b, Mechanism of cell cycle control. Abbreviation: IFN-γ, Interferon gamma; IFNGR1/2, Interferon gamma receptor 1/2; JAK1/2/3; Janus kinase 1/2/3; STAT1, signal transducer and activator of transcription protein family, MDM2, E3 ubiquitin-protein ligase Mdm2; MDM4, MDM4, P53 Regulator; TP53, Tumor Protein P53, CDKN1A, Cyclin Dependent Kinase Inhibitor 1A (p21, Cip1); CDKN1B, Cyclin-Dependent Kinase Inhibitor 1B (P27, Kip1); CDK2, Cyclin Dependent Kinase 2; CCNE1, G1/S-Specific Cyclin-E1; CDKN2A, Cyclin-Dependent Kinase Inhibitor 2A/ZB (p16^{lnk4a}/p14^{Arf}); CDKN2C, Cyclin-Dependent Kinase Inhibitor 2C (p18, inhibits CDK4) ; CDK4, Cyclin Dependent Kinase 4; CDK6, Cyclin Dependent Kinase 6; CCNDI, Cyclin D1; CCND2, Cyclin D2, CCND3, Cyclin D3.

### EXAMPLES

### 1. Introduction

Cancer immune-control largely eliminates cancers through cytotoxic responses¹⁻⁵, but additional mechanisms are needed to protect against cancer cells that escape from cytotoxic immune responses⁶⁻⁸. As cytokine-induced senescence (CIS) can stably cancer cell proliferation¹⁰, the inventors asked whether cancer immune-control requires senescence-inducing cell cycle-regulators to arrest those cancer cells that survive cytotoxic immune responses. Here, the inventors show in mice that natural and therapy-induced immune responses largely deleted cancers but induced senescence in those cancer cells that escaped from elimination. Cancer control required senescence induction, as cancers deficient in p16^{lnk4a} or p21^{Cip1} showed uncontrolled growth despite preserved susceptibility to apoptosis and cytolysis. Induction of senescence and cancer immune-control required T cells and activation of p16^{lnk4a} and p21^{Cip1} by the interferon (IFN)-g-STAT1 signalling cascade. In line with this, metastatic human melanomas that progressed within < 3 months during immune checkpoint blockade (ICB) had losses of senescence-inducing genes and ≥ 3fold amplifications of senescence inhibitors, were resistant to CIS but susceptible to apoptosis. Such mutations were infrequent in metastatic melanomas that regressed during ICB for > 1 year. Thus, cancer immune-control required the IFN-dependent activation of cancer-intrinsic, senescence-inducing cell cycle regulators, as second mechanism to stably arrest those cancer cells that escaped from eradication.

### 2. Material and Methods

### Animals

C3HeB/FeJ mice (C3H) were purchased from The Jackson Laboratory (Bar Harbor, ME, USA). Syngeneic transgenic TCR2 mice³² express a T cell receptor (TCR) specific for Tag peptide 362-384 on CD4⁺ T cells, RIP-Tag2 (RT2) mice express the T antigen under control of the rat insulin promotor (RIP) that leads to pancreatic islet cancers (RT2-cancers)^{10,33} and double transgenic RT2.*Stat1*^{*-*/*-*} mice (backcross of 129S6/SvEv-*Stat1^{tm1Rds}* mice³⁴) were provided by Taconic and backcrossed to C3H¹⁰. All animals were bred under specific pathogen-free conditions. *λ-MVC* mice and double transgenic λ*-MYC.p21*^{*-*/*-*} (both C57BL/6 background) express a human *MYC* oncogene under the control of the immunoglobulin I enhancer and develop endogenous B-cell lymphoma³¹. Mice with C3HeB/FeJ background were bred in the animal facility Tübingen. Mice with C57BL/6 background were bred in the animal facility Munich. Animal experiments were in accordance with animal welfare regulations and had been approved by the local authorities (Regierung von Oberbayern and Regierungspräsidium Tübingen).

### Treatment of RT2 cancers in C3H mice

A total of 1 × 10⁶ cells (in 100 µl NaCl) RT2-, RT2.*Stat1*^{-/-}, RT2.p16-/- or RT2.^{p16CRISP-Cas} cancer cells were s.c. transplanted, three days after depletion of CD8 cells with 100 µg anti-CD8 mAb (Rm-CD8-2 AK, Core Facility mAb, Helmholtz-Zentrum Münch). CD8 depletion cells was repeated every ten days till the tumor lesion became palpable. Once tumors were ≥ 3 mm, mice were treated with anti-PD-L1mAb plus anti-LAG-3 mAb once per week (initially 500 µg, then 200 µg). Ctr mice received isotype-control antibodies (clone LTF-2 and HRPN, Bio X Cell). Tumor growth was monitored using a calliper and blood glucose was measured twice a week using an Accu-Check sensor for up to 8 weeks. Mice were sacrificed after 4 treatment cycles, when the tumor diameter reached > 15 mm or when blood glucose dropped the second time below 30 mg dl⁻². Tumors were collected for ex *vivo* analysis.

### Treatment of RT2 or RT2.Stat1^{-/}⁻ mice

Ten- to eleven-week-old old female RIP-Tag2 (RT2) or RT2.*Stat1*^{-/-} mice were irradiated with 2 Gy one day before the first i.p. transfer of 1 × 10⁷ tumor antigen-specific T_{H}1 cells (TAA-T_{H}1, isolated from female TCR2 mice and generated as described earlier³⁵). Cell transfer was applied once weekly. Anti-PD-L1 mAb (clone 10F.9G2, Bio X Cell) plus anti-LAG-3 mAb (clone C9B7W, Bio X Cell) were i.p. injected twice per week (initially 500 µg each, afterwards 200 µg). Ctr mice received isotype-matched control antibodies (clone LTF-2 and HRPN, Bio X Cell) and PBS. Blood glucose was measured twice per week using the HemoCue Glucose 201+ System (HemoCue). Treatment was ended either after 4 treatment cycles for ex *vivo* analysis of tumor tissue, or when the blood glucose of the mice dropped twice below 30 mg dl⁻¹ or when disease reached evidence; mice had no food restriction.

### Treatment of λ-MYC mice

λ-MYC mice and double transgenic λ-*MYC.p21*^{-/-} mice received intraperitoneal (i.p.) injections of 100 µg anti-PD-1 mAb (clone J43, Bio X Cell) and 100 µg anti-CTLA-4 mAb (clone UC10-4B9, BioLegend) (ICB) two to four times every ten days, starting at day 55 after birth. Control mice (Ctr) received no treatment. For IFN-γ neutralization (ICB/anti-IFN-y mAb), mice were additionally treated with an initial dose of 500 µg on day 54 and later with 300 µg anti-IFN-γ mAb (clone XMG1.2³⁵, Core Facility mAb, Helmholtz-Zentrum München) 6 h prior to the anti-PD-1/CTLA-4 mAbs injection. Administration of 100 µg of the IFN-γ-neutralizing mAb was continued at ten day intervals until the mice developed lymphomas. T cells were depleted with CCC µg YYY mAb, injected on day X, B, D. Mice were sacrificed as soon as tumors became clinically visible.

### Immunofluorescence staining

Immunofluorescence analyses were performed essentially as previously described¹⁰. In detail, fresh frozen 5 µm serial cryosections of lymph nodes from *λ-MVC* mice, whole pancreata from RT2- and RT2.*Stat1*^{-/-} mice or isolated RT2-, *Stat1-,* or p16^{lnk4a}-deficient RT2-cancer cells were fixed with perjodate-lysine-paraformaldehyde. Sections were blocked using donkey serum and incubated with rabbit-anti-p16^{lnk4a} (Serotec), rat-anti-Ki67 (eBioscience), rat-anti-p21^{Cip1} (Abcam), goat-anti-CD20 (Santa Cruz), rabbit-anti-CD3 (DCS), rabbit-anti-pHP1γ phospho S93 (Abcam) and rabbit-anti-H3K9me3 (Abcam). Bound antibodies were visualized using donkey-anti-rabbit-Cy3 (Dianova), donkey-anti-rat-Alexa 647 (Dianova), donkey-anti-rat-Cy3, donkey-anti-goat-Cy3 and donkey-anti-rabbit-Alexa 488. For nuclear staining 4500 Yopro (Invitrogen) or DAPI (Sigma) was used. Sections were analysed using a LSM 800 confocal laser scanning microscope (Zeiss Oberkochen). Images were processed with the software ZEN 2.3 (blue edition) and the Image Analysis Module.

### Quantification of immunofluorescence

Tumor areas of images derived from three individual stained cryosections per tissue sample were analysed. For quantification of the Ki67 staining, 4500 Yopro stained nuclei were counted for Ki67⁺ cells (1500 per slide). For quantification of p16^{lnk4a}, the area (µm²) of the nuclear p16^{lnk4a} signal derived from 4500 Yopro stained nuclei (1500 per slide) was measured.

### Hematoxylin and eosin (HE) staining

HE staining of the serial cryosections was performed according to standard procedures.

### SA-β-gal detection

20 µm serial cryosections were fixed in 2% formaldehyde/ 0.25% glutaraldehyde and washed in PBS/MgCl₂. Slides were incubated in X-gal staining solution (5-bromo-4-chloro-3-indolyl-beta-D-galactopyranoside). The stained slides were rinsed in PBS/MgCl₂ and analysed using a Nikon Eclipse 80i Microscope; magnification × 4. Each tissue sample was stained at a pH of 4.0, 5.5, and 7.0.

### SA- β-gal positive percentage quotation with Adobe Photoshop CS6

All images were analysed with the tool white balance to obtain the same white background (*White Balance Tool*), followed by *Quick Mask Mode* to identify only the tumour area. Using this area the arithmetic mean of the blue-green values was obtained by the filter *Blur Average Tool. The Eye Dropper Tool* was used to identify the RGB colour code to get the corresponding colour field. Afterwards the tumour area was reselected using the previous created *Quick Mask Mode Layer.* The pixels outside the tumour area were deleted by inversing the selection. The number of pixels in the Histogram correlates with the tumour area. *The Posterize Tool* separated the different tonal values, in our case blue-green. *The Magic Wand Tool* was used to select and delete the white pixels. The number of pixels correlates with the area of β-gal stained tumour cells, the quantification of β-gal stained tumour cells were calculated in percentage.

### Detection of SA-β-gal activity for Electron Microscopy

Small tissue samples (semi-thin 0.5 µm and for EM ultra-thin 20 nm, respectively) were fixed in fixation solution (0.25% Glutaraldehyde in 2% PFA) and washed in PBS/MgCl₂ solution. X-Gal staining solution was added for 12 h. Samples were washed in PBS/MgCl₂ solution afterwards followed by Karnovsky fixation. Samples were embedded in Glycid ether for electron microscopy analysis.

### Electron Microscopy

Tissues were fixed with Karnovsky's fixative for 24 h. Post-fixation was based on 1% osmium tetroxide containing 1.5% K-ferrocyanide in cacodylate buffer. After following standard methods, blocks were embedded in glycide ether and cut using an ultra-microtome (Ultracut, Reichert, Vienna, Austria). Ultra-thin sections (30 nm) were mounted on copper grids and analysed using a Zeiss LIBRA 120 transmission electron microscope (Carl Zeiss, Oberkochen, Germany) operating at 120 kV.

### Cell culture

Adherent RT2-cancer cells were cultured in Dulbecco's Modified Eagle's Medium (DMEM) supplemented with 10% foetal calf serum (FCS), nonessential amino acids, sodium pyruvate, antibiotics, and 50 µM 2-mercaptoethanol at 37°C and 7.5% CO₂. The murine melanoma cell lines B16 and B16-OVA were cultured in DMEM medium, containing 10% FCS and penicillin/streptomycin (100 U ml⁻¹; all from Biochrom AG), at 37°C and 5% CO₂. The human TüMel and patient-derived xenograft (PDX) cell lines were cultured in RPMI-1640 Medium supplemented with 10% FCS, nonessential amino acids, sodium pyruvate, antibiotics, and 5 µg ml⁻¹ Plasmocin^{™} (Invitrogen) at 37°C and 5.0% CO₂.

### Generation of primary murine RT2- or Stat1^{-/}⁻ cell lines

RT2-cancers were isolated from the pancreas of female RT2 or RT2.*Stat1*^{-/-} mice by collagenase digestion (1 mg ml⁻¹, Serva, Heidelberg, Germany) as described^{10, 35}. Briefly, whole encapsulated tumors were separated under a dissection microscope (Leica Microsystems) and further processed for immunofluorescence microscopy, immunohistochemistry or gene expression analysis. Alternatively, single tumor cells were obtained by incubation of the tumors in 0.05% trypsin/EDTA solution (Invitrogen) at 37°C for 10 min. After incubation, RT2-cancer cells were seeded onto tissue culture plates.

### Generation of primary p16^{lnk4a}-deficient RT2-cell lines

CDKN2a-loss variants on chromosome 4, qC4.A were generated by random selection from CIS- or ICB- resistant RT2-cancer cells.

### Generation of CRISPR-mediated deletion of p16^{lnk4a} in primary RT2-cell lines

gRNAs targeting Exon 2 (position 120-142 and 125-157) of murine Cdkn2a^{lnk4a} (sequence from Ensembl genome browser 97) were designed using CRISPRdirect³⁶. The gRNAs were cloned into pSpCas9(BB)-2A-GFP (pX458; Addgene plasmid ID: 48138) using Bbsl restrictions sites³⁷ (gCdkn2a_1: 5'-gcg tcg tgg tgg tcg cac agg-3' (SEQ ID No. 1), gCdkn2a_2: 5'-gac acg ctg gtg gtg ctg cac-3' (SEQ ID No. 2)). The DNA oligos were ordered from Sigma Aldrich and annealed according to the Zhang protocol. The annealed oligos were cloned into pSpCas9(BB)-2A-GFP (pX458; Addgene plasmid ID: 48138) using Bbsl restrictions sites. MaxiPreps of plasmids were done using the Qiagen EndoFreeMaxi Kit. The primary RT2-cell lines were transfected using the Quiagene Effectene Transfection Reagent.

### Generation of primary human melanoma cell lines

Patient tissue samples were either directly digested (TüMel) or expanded in a PDX mouse model by implanting the digested tumor tissue subcutaneously, as described previously^{38, 39}.

### Senescence assay

RT2, RT2.*Stat1*^{-/-}, RT2*.p16*^{lnk4a}, RT2-CRISPER-control or RT-CRISPER-*p16*^{lnk4a} cancer or human melanoma-derived (PDX) cells were cultivated with medium containing 100 ng ml⁻¹ IFN-γ and 10 ng ml⁻¹ TNF or exclusively with medium (Ctr) for 96 h, washed, then cultivated with medium for another 4 - 6 days (till to confluence of the Ctr cells) and counted¹⁰.

### SA-β-galactosidase activity assay in vitro

After treatment with IFN-γ and TNF, RT2-cancer cells were fixed for 15 min at room temperature, and then stained for 16 h at 37°C using the β-galactosidase staining kit (United States Biological) as previously described¹⁰. In addition, cell nuclei were stained with 4',6-diamidin-2-phenylindole (DAPI; Invitrogen). SA-β-gal-positive and - negative cells were counted using a Zeiss Axiovert 200 microscope equipped with Visiview software and analysed by using imaged software (NIH).

### Apoptose assay

For apoptosis induction, RT2-cancer cells were exposed to etoposide (Eto, 100 µM, Bristol-Meyers-Squibb, ETOPOPHOS^{®}), staurosporine (Sta, 0.5 µM, BioVision) or medium (Ctr) for 24 h and stained for propidium iodide and Annexin V. Positive cells were detected by flow cytometry analysis.

### Chromium release assay

CTL-mediated cytotoxicity was measured by chromium release as previously described¹⁰. Briefly, 2.5 × 10⁶ target cells were labelled with 250 µCi (9.25 MBq) ⁵¹NaCr (Hartmann Analytic) at 37°C for 1.5 h. B16-OVA cells were used as positive controls, B16-F10 melanoma cells were used as negative controls.

### Patients and specimen collection

Patients with metastatic melanoma were treated with either anti-PD-1 mAb or combined anti-PD-1 and anti-CTLA-4 mAb. Non-responders were 30 patients (43.3 % female, 61.5 median age, 22 - 89 age range) that progressed during the first 3 month of therapy. Responders were 12 patients (33.3% female, 56.5 median age, 27 - 75 age range) that had either a partial (> 30%) or complete tumor regression over more than 1 year. Tumor biopsies were compared to healthy tissue from the safety margins as control tissue. Ethical approval was obtained from the Ethics Committee Tübingen. All patients had signed the written informed consent form for research analyses. The study was carried out in accordance with the Declaration of Helsinki and good clinical practice.

### Sequencing

All patient samples were analysed using a hybridization-based custom gene panel. Since the patient samples were collected in clinical routine, three different versions of the panel were used (ssSCv2, ssSCv3 and ssSCv4). The number of target genes was increased from one version to the next starting from 337 genes on ssSCv2, to 678 genes on version ssSCv3 and 693 on the current version ssSCv4. The panels were designed to detect somatic mutations (SNVs), small insertions and deletions (INDELs), copy number alterations (CNAs) and selected structural rearrangements Supplemental Data Table 1-3 (custom gene panel ssSCv2 ssSCv3 ssSCv4). The library preparation and in solution capture of the target region was performed using the Agilent SureSelectXT and SureSelectXT HS reagent kit (Agilent, Santa Clara, CA). DNA from tumor (FFPE) and matched normal controls (blood) were sequenced in parallel on an Illumina NextSeq500 using 75bp paired-end reads. The tumor samples were sequenced to an average sequencing depth of coverage of 511x and the normal control samples to 521x, respectively. An in-house developed pipline, called "megSAP" was used for data analysis (https://github.com/imgag/megSAP, version: 0.1-733-g19bde95 and 0.1-751-g1c381e5). In brief, sequencing reads were aligned to the human genome reference sequence (GRCh37) using BWA (vers. 0.7.15)⁴³. Variants were called using Strelka2 (vers. 2.7.1) and annotated with SNPeff/SnpSift (vers. 4.3i)^{44, 45}. The overall mutational rate was as described previously⁴⁶. For validity and clinical relevance, an allele fraction of ≥ 5% (i.e. ≥ 10% affected tumor cell fraction) was required for reported mutations (SNVs, INDELs). Copy number alterations (CNAs) were identified using ClinCNV (unpublished, https://github.com/imgag/ClinCNV), a method for multi-sample CNV detection using targeted or whole-genome NGS data. In brief, the method consists of four steps: i) quantification of reads per target region, ii) normalisation by GC-content, library size and median-coverage within a cohort of samples sequenced with the same NGS panel, iii) calculation of log2-fold changes between tumor and normal sample, iv) segmentation and CNV calling. Using log2-fold changes ClinCNV estimates statistical models for different copy number states per region (conditioned by tumor sample purity) and reports a likelihood for each statistical model, assuming that the majority of samples are diploid at a focal region. The log-likelihood of the diploid model is subtracted from alternative models, resulting in positive likelihoods for true alternative copy number states. Finally, maximum segments of contiguous regions with positive log-likelihood ratios are identified in an iterative manner. Segments consisting of at least three regions with log-likelihood ratio ≥ 40 and CN state ≤ 1.5 or ≥ 3 are reported as CNVs. Quality control (QC) parameters were collected during all analysis steps⁴⁷.

### Comparative genomic hybridization (CGH) array

DNA was isolated from RT2-cancer cells or reference spleen (wildtype male) tissue with the DNeasy Blood & Tissue Kit (Qiagen) according to the manufacturer's instructions. DNA was labelled using the SureTag Complete DNA Labelling Kit (Agilent Technologies) and hybridized on an Agilent Mouse Genome CGH Microarray, 2x105K (Agilent Technologies) and the image was analysed using Feature Extraction 10.5.1.1 and Agilent Genomic Workbench Lite Edition 6.5 with Genome Reference Consortium Mouse Build 38.

### Quantitative PCR

RT2-cancer cells were harvested by trypsin digestion and snap frozen in liquid nitrogen. RNA was prepared using the Nucleospin RNA Mini kit (Macherey-Nagel GmbH & Co. KG); cells were lysed using Tris(2-carboxyethyl)phosphine (TCEP)-containing RL1 buffer, followed by DNase digestion (Invitrogen Corp). RNA quality was controlled by agarose gel electrophoresis and by OD600 measurements using a photometer (Eppendorf AG). Complementary DNA was prepared using the iScript cDNA synthesis kit (Bio-Rad Laboratories GmbH). Quantitative PCR was performed with SybrGreen using a LightCycler 480 (Roche). Reference genes were evaluated using geNorm⁴², and gene expression was analysed using qbase software (Biogazelle) based on the delta-delta-CT-method. The following primers were used: *SV40-Tag* sense 5'-tccactccacaattctgctct-3' (SEQ ID No. 3), antisense 5'-ttgcttcttatgttaatttggtacaga-3 (SEQ ID No. 4); *Cdkn2a* sense 5'-ttgcccatcatcatcacct-3' (SEQ ID No. 5), antisense 5'-gggttttcttggtgaagttcg-3' (SEQ ID No. 6); *Actb* sense 5'-ctaaggccaaccgtgaaaag-3' (SEQ ID No. 7), antisense 5'-accagaggcatacagggaca-3' (SEQ ID No. 8); *Eef1a1* sense 5'-acacgtagattccggcaagt-3' (SEQ ID No. 9), antisense 5'-aggagccctttcccatctc-3' (SEQ ID No. 10).

### Magnetic resonance imaging

Imaging was performed with ten-week-old RT2 mice under 1.5% isoflurane anaesthesia using a 7T small animal magnetic resonance scanner (ClinScan, Bruker Biospin MRI GmbH, Ettlingen, Germany) as described⁴⁰.

### Light sheet fluorescence microscopy (LSFM)

Five-week-old RT2 or RT2.*Stat1*^{-/-} mice were treated with TAA-T_{H}1 cells or NaCl as described. Mice were injected i.v. with 50 µg of Alexa Fluor^{®} 700 anti-mouse CD4 mAb clone GK1.5 and Alexa Fluor^{®} 647 anti-mouse CD11b mAb clone M1/70 (BioLegend), 48 h after the second treatment. After 2 h, organs were harvested and fixed in 4% paraformaldehyde/PBS solution at 4°C for 8 h. Tissue was dehydrated at room temperature using increasing concentrations of ethanol (30, 50, 70, 80, 90%) for 2 h each and in 100% ethanol at 4°C overnight. Tissues were incubated in n-hexane for 2 h and then cleared using two parts benzyl benzoate and one part benzyl alcohol (Sigma-Aldrich) three times for 30 minutes. Air exposure was strictly avoided during this step. The samples were then visualized and analysed using a custom-built laser scanning light sheet microscope using a high NA 20 × magnification⁴¹ and reconstructed using the IMARIS software (Bitplane).

### Flow cytometry

Five-week-old RT2 or RT2.*Stat1*^{-/-} mice were treated with TAA-T_{H}1 cells or NaCl as described³⁵, and 48 h after the second treatment mice were sacrificed. The pancreatic lymph node was separated from the pancreas tissue of each mouse, the pancreas was homogenized via a 200 µm cell strainer in DMEM media containing 20% FCS at 4°C, erythrocytes were lysed with ACK lysis buffer (Cambrex), and samples were stained with fluorochrome-conjugated antibodies (anti-mouse CD4-Pacific Blue, clone GK1.5; anti-mouse CD8a-PE-Cy7, clone 53-6.7; anti-mouse CD45.2-APC-Cy7, clone 104; anti-mouse-CD11c-APC, clone N418; anti-mouse CD11b-Pacific blue, clone M1/70 (BioLegend)). Cells were separated again via a 50 µm cell strainer. Flow cytometry was performed using a FACS Aria and analysed with DIVA software (Becton Dickinson). Alternatively flow cytometry analyses with T_{H}1 cells or RT2 cancer cells were stained with fluorochrome-conjugated antibodies (anti-mouse-PE IFN-γ, anti-mouse-PE TNF-α, anti-mouse-PE IL-4, anti-mouse- PE β2-microglobulin; anti-mouse PE/Cy7 CD274 (B7-H1, PD-L1) (BioLegend)) were performed on a LSRII cytometer (BD Bioscience) and analysed by FlowJo software version 10.

### Statistical analysis

The experiments were not randomized. The investigators were not blinded to allocation during the experiments or outcome assessment. No power calculations were used, but sample sizes were selected on the basis of previous experiments; *in vitro* results were based on three independent experiments to guarantee reproducibility of findings. The statistics software JMP version 12.2.0 (SAS Institute) and GraphPad Prism version 6 (GraphPadSoftware, Inc., California, USA) were used for statistical analyses and for the generation of diagrams. To address the question whether the treatment effect was different between two genotypes, the decadic logarithms of tumour volumes were analysed in ANCOVAs, using the nominal factors "mouselD" (nested under "treatment" and "genotype"), "treatment" and "genotype" as well as the combination "treatment" and "genotype"; "time" was used as continuous factor; finally, the combinations of "mouse ID" and "time", "treatment" and "time", "genotype" and time" and the most important combination "treatment" and "genotype" and "time" were used. For purpose of normalization, decadic logarithms of tumour volume were used in the analyses; zero observations were replaced by half the minimum of positive values before calculating the logarithms. Group comparisons were made with non-parametric, unpaired, two-tailed Mann-Whitney (Wilcoxon) tests or parametric, unpaired, two-tailed t test with Welch's correction for unequal variances. Log-rank test was used for the comparison of survival from I-MYC mice, RT2 mice and tumor latency curves. Because of disparate censoring between RT2 mice and RT2.*Stat1*^{-/-} mice Fisher's exact tests was used to compare the survival (as indicated in the text). *N* refers to the number of patients, mice or samples and cell lines from different mice, respectively.

### 3. Results

Natural immune responses and immunotherapy with ICB revealed that tumor-infiltrating cytotoxic T cells and natural killer cells can be activated to cause cancer regression through cytolysis and apoptosis in humans. Yet, cancer cells are often not completely eliminated and survive in a poorly defined, but controlled state of dormancy^{2, 4, 11-14}. Loss of dormancy is a major cause of treatment resistance that may result from inappropriate immune activation, low numbers of tumor-associated copy number variants (CNVs), resistance to lysis or apoptosis, and unknown mechanisms^{2, 6, 11-13, 15-18}. An increasing number of data associates melanoma progression and treatment resistance of cancers with functional losses of the IFN-JAK1-STAT1 signalling pathway^{11, 12, 14, 16, 19-23}. As IFN-JAK1-STAT1 signalling activates two key-inducers of senescence, p16^{lnk4a} and p21^{Cip1 10, 22, 24-27}, the inventors asked whether cancer immune-control requires induction of the tumor-intrinsic senescence-inducing p16^{lnk4a}-CDK4/6-Rb1 and MDM-p53-p21^{Cip1} cell cycle regulators to arrest those cancer cells that escape from cytotoxicity.

In vitro activation of p16^{lnk4a} and p21^{Cip1} requires IFN-g-signalling in the tumor cells^{ref}. The inventors therefore first asked whether *in vivo* activation of p16^{lnk4a}, senescence induction and cancer immune-control also require a functioning IFN-g-signalling cascade in the cancer cells. To address this, the inventors implanted either *Stat1*-positive or *Stat1*-negative cancer cells from RIP-Tag2 mice, where expression of the T antigen under the control of the rat insulin promoter (RIP) leads to pancreatic islet cancers (RT2-cancers)¹⁰, into syngeneic mice. Most STAT1-positive and -negative RT2-cancers (> 80%) were rejected. Yet, following CD8-depletion the tumors grew and had a proliferative Ki67⁺p16^{lnk4a-} phenotype (Fig. 3a, b).

To determine whether the immune system can still control these cancers, the inventors stimulated the immune system by ICB once tumor diameters reached ≥ 3 mm. The *Stat1*-positive RT2-cancers became growth arrested or regressed and the few remaining cancer cells displayed a senescent p16^{lnk4a+}, Ki67⁻ phenotype that was also positive for phosphorylated heterochromatin protein 1g (S93) (pHP1g) in senescence-associated heterochromatin foci (SAHF), H3K9me3 and senescence-associated b-galactosidase (SA-b-gal) (Fig. 3a; Fig. 11a-c), (Fig. 3a). In contrast, *Stat1*-negative RT2-cancers grew rapidly with and without ICB. The tumor cells were p16^{lnk4a-}, expressed Ki67 but neither pHP1g⁻, H3K9me3⁻, nor SA-b-gal⁻ (Fig. 3b).

As STAT1 was not required for tumor elimination by CD8⁺ cells but for the induction of p16^{lnk4a} and an efficient cancer immune control, the data suggest that cancer immune-control required activation of the tumor cell intrinsic, senescence-inducing cell cycle regulator p16^{lnk4a} to control those cancer cells that were not rejected. To address this question, the inventors generated p16^{lnk4a}-deficient RT2-cancer cell lines with loss of the p16^{lnk4a} gene locus (*Cdkn2a*) through *in vitro* and *in vivo* selection. Comparative genomic hybridization showed a loss of p16^{lnk4a} on chromosome 4 (qC4.A) as the only genetic aberration common to all six cell lines (Fig. 10a). PCR-analysis confirmed the loss of p16^{lnk4a} expression (Fig. 10b). While the parental line was susceptible to IFN-g- and TNF-induced senescence and to apoptosis (Fig. 3a, c), the *CDKN2a*-loss mutant cell lines were resistant to CIS but susceptible to apoptosis *in vitro* (Fig. 3d). To test the role of p16^{lnk4a} for tumor control *in vivo,* the inventors injected the tumors into syngeneic mice and started ICB once tumors reached a diameter of 3 mm. While ICB efficiently controlled the p16^{lnk4a}-expressing RT2-cancers and induced a senescent phenotype that was Ki67⁻ but positive for p16^{lnk4a}, pHP1g, H3K9me3 and SA-b-gal (Fig. 3a; Fig. 11a-c), all p16^{lnk4a}-deficient RT2-cancer lines rapidly grew in the absence or presence of ICB-therapy. Importantly, ICB failed to induce senescence in p16^{lnk4a}-deficient RT2-cancers as they displayed a Ki67⁺p16^{lnk4a-}, pHP1g⁻, H3K9me3⁻, SA-b-gal⁻ phenotype (Fig. 3e; Fig. 11a-c). To test whether this resistance to ICB specifically resulted from the p16^{lnk4a} loss, the inventors deleted p16^{lnk4a} (gCdkn2a) using CRISPR-Cas9. *In vitro* RT2-cancer cells transfected with either a control sgGFP or the gCdkn2a construct grew with similar dynamics. Both cell types were sensitive to apoptosis, while the p16^{lnk4a}-deficient cells were resistant to CIS (Fig. 12a, b). All CRISPR-Cas9 control cell lines were rejected by the CD8-depleted mice. In contrast, 80 % of the CRISPR-Cas9-mediated p16^{lnk4a}-deficient RT2-cancer cell lines grew in syngeneic mice (Fig. 12c). Again, ICB did not control the growth of these p16^{lnk4a}-deficient RT2-cancers that all showed a Ki67⁺, p16^{lnk4a}, pHP1g⁻, H3K9me3⁻, SA-β-gal- proliferative phenotype (Fig. 3f; Fig. 11c). Thus, cytotoxic immune responses can directly reject also p16^{lnk4a}-deficient RT2-cancers, but senescence-induction through IFN-g-mediated activation of the cell cycle regulator p16^{lnk4a} was strictly needed to control those cancer cells that survived the cytotoxic immune responses.

To determine whether IFN-signalling is also needed for the induction of p16^{lnk4a}, p21^{Cip1}, senescence and the control of endogenous cancers that are destroyed by strong T cell responses, the inventors treated RT2-mice with a major cancer load, as documented by magnetic resonance imaging (Fig. 6). Four weeks prior to the expected death the inventors treated mice by the combination of anti-LAG-3/anti-PD-L1 mAb and adoptive T cell transfer (AT), the most efficient ICB combination that largely eradicates all cancer cells²⁹. Indeed, combined ICB/AT-therapy destroyed the cancers, significantly decreased the islet size within 4 weeks (p < 0.05), prolonged life of RT2-mice and functionally restored the blood glucose control (Fig. 2a-c). While ICB/AT largely destroyed the RT2-cancers, it failed to eradicate all cancer cells (Fig. 2b-d; Fig. 7a-d). The remaining cancer cells showed a senescence phenotype as they expressed p16^{lnk4a}, p21^{Cip1}, H3K9me3, pHP1g, and SA-b-gal but were Ki67⁻ (Fig. 2d, e; Fig. 7a-d). Electron microscopy confirmed the nuclear accumulation of SA-b-gal in the senescent tumor cells (Fig. 7e). At this advanced stage the combined ICB/AT-therapy was superior to either ICB or AT monotherapy that provided intermediate results (Fig. 2a-e; Fig. 7a-d). Sham-treated mice showed large tumors (Fig. 2b), strongly enriched in Ki67⁺ RT2-cancer cells that were negative for p16^{lnk4a}, p21^{Cip1}, H3K9me3, pHP1g, and SA-b-gal (Fig. 2d, e; Fig. 7a-d). To determine whether p16^{lnk4a} and p21^{Cip1} induction and cancer control by the combined ICB/AT required an intact IFN-signalling pathway, we investigated combined ICB/AT in RT2.*Stat1*^{-/-} mice. Even combined, ICB/AT induced neither p16^{lnk4a} nor p21^{Cip1} and did not reduce the cancer size, prolong lifetime or delay cancer progression in *Stat1*-deficient mice (Fig. 2a-c). Three-dimensional imaging and FACS analyses revealed that the adoptively transferred T cells and dendritic cells infiltrated RT2-cancers of *Stat1*^{*-*/*-*} or *Stat1*^{*+*/*+*} mice with similar dynamics (Fig. 8a-e). In vitro, *Stat1*^{*-*/*-*} cancer cells were fully susceptible to apoptosis or T cell-mediated killing but were resistant to CIS (Fig. 9a-d). *Stat1*-deficient cancer cells were also *in vivo* resistant to CIS, as cancer cells of *Stat1-*deficient mice remained Ki67⁺ and negative for p16^{lnk4a}, p21^{Cip1}, H3K9me3, pHP1g, or SA-b-gal activity despite immune activation by combined ICB/AT (Fig. 2d, e; Fig. 7a-d). Thus, even the ICB/AT combination failed to eradicate all cancer cells; instead it induced a *Stat1*-dependent, p16^{lnk4a} and p21^{Cip1} expressing, senescence phenotype in the surviving cancer cells. As *Stat1*-deficient cancer cells were normally susceptible to T cell mediated cytolysis and apoptosis, the data support the concept that cancer immune-control required STAT1-mediated activation of the cell cycle regulators p16^{lnk4a} and p21^{Cip1} in the tumor cells, even if tumors were largely destroyed by strong cytotoxic immune responses.

As *Stat1*-deficient cancers expressed neither p21^{Cip1} nor p16^{lnk4a}, cancer immune-control may also require p21^{Cip1} activation. To test the need of senescence-inducing p21^{Cip1} for cancer immune-control and to determine whether senescence induction is also needed for other tumors than RT2-cancers, the inventors studied the role of p21^{Cip1} in the immune control of lymphomas. The inventors studied I-*MYC* mice, where a human *MYC* oncogene under the control of the immunoglobulin I enhancer induces the development of endogenous B-cell lymphomas. Untreated mice died within < 150 days (Fig. 1a) from Ki67⁺p16^{lnk4a-}, CD20^{low} B-cell lymphomas that destroyed lymph nodes and spleen (Fig. 1b, c; Fig. 5a). The Ki67⁺ B cells were also negative for p21^{Cip1}, pHP1g, H3K9me3 or SA-b-gal, showing that the tumors had a high proliferative capacity and were not senescent (Fig. 1b; Fig. 5b-d). Surprisingly, combined ICB with anti-CTLA-4 and anti-PD-1 mAb protected 20%-40% of I-*MYC* mice from lymphomas, as they were still healthy at > 250 days (Fig. 1a), a lifetime that has never been achieved by any other therapy in this mouse model. Treatment with either mAb alone did not rescue mice from lymphomas. Lymph nodes from healthy ICB-treated I-*MYC* mice showed a normal architecture with normal B and T cell areas, no T cell infiltration and no destruction, even though the protection from lymphomas was strictly T cell-dependent (Fig. 1b/3bNEW; Fig. 5a). Nodal B cells from ICB-treated, healthy I-*MYC* mice were Ki67⁻ but strongly expressed nuclear p16^{lnk4a} and p21^{Cip1} (Fig. 1b, c), pHP1g, H3K9me3 and SA-b-gal (Fig. 5b-d), displaying a senescent phenotype26. This suggests that ICB-mediated protection from B-cell lymphomas included mechanisms other than cancer cell killing. To test whether !CB-mediated lymphoma prevention required the senescence-inducing p21^{Cip1}, the inventors generated syngeneic I-*MYC.*p21^{Cip-/-} mice. These mice developed lymphomas with slower dynamics than I-*MYC* controls (Fig. 1d). In I-*MYC*.p21^{CiP-/-} mice ICB failed to significantly delay death and all mice died from lymphomas (Fig. 1d). Histology revealed lymph node destruction by Ki67⁺ CD20^{low} B cells (Fig. 1b; Fig. 5a) that were devoid of the senescence markers p16^{lnk4a}, p21^{Cip1}, pHP1g, H3K9me3, or SA-b-Gal (Fig. 1b, c; Fig. 5b-d). ICB-mediated p21^{Cip1}-induction and lymphoma prevention strictly required IFN-g, as mice receiving ICB in the presence of anti-IFN-g monoclonal antibodies (mAb) died from CD20^{low} B-cell lymphomas as fast as untreated mice (Fig. 1a). B cells were Ki67⁺ and negative for p21^{Cip1} and for p16^{lnk4a} (Fig. 1b, c), pHP1g, H3K9me3 or SA-b-Gal (Fig. 5b-d), showing that IFN-g was needed to activate the cell cycle regulators p16^{lnk4a} and p21^{Cip1} molecules that, in turn, were required for senescence-induction *in vivo.* Thus, ICB-mediated immune activation strictly required the IFN-g-dependent activation of the senescence-inducing cell cycle regulator p21^{Cip1} in the I-*MYC* B cells to prevent the transition of pre-malignant B cells into B-cell lymphomas.

ICB is an approved standard of care therapy for metastatic melanoma and some other cancers, and efficient in about 40% of patients with metastatic melanoma. Another 40% are non-responders that often progress rapidly despite ICB-therapy.^{11, 12, 14, 16, 19-23, 30}. Based on the experimental data, the inventors asked whether cell cycle regulator genes that control senescence induction were also needed for cancer immune-control in humans. To address this, the inventors compared the genetic alterations by targeted panel sequencing of 30 melanomas from consecutive non-responders, where metastases progressed within < 3 months of ICB with the genetic alterations of 12 responders, where metastases regressed during ICB ≥ 1 year. In agreement with published data responder patients had a significantly higher tumor mutational burden than non-responders¹⁵ (Fig. 4a). Based on the preclinical *in vivo* data, the inventors particularly investigated senescence-associated genes. The inventors focused on somatic alterations, namely CNVs and single nucleotide variants (SNVs) in key cell cycle control genes (*CCND1*/*2*/*3; CDKN2A*/*B*/*C, CDK4*/*6, CCNE1, CDKN1A*/*B, RB1, TP53, MDM214),* as well as *JAK1,2,3* and *MYC.* Both groups had a similar distribution of genetic aberrations in *JAK1,2,3, MYC* and the cell cycle control genes when all somatic alterations (SNVs and CNVs) were included (Fig. 4b). In contrast, comparing the number of fully inactivating mutations (homozygous deletions and loss of heterozygosity (LOH)) or amplifications ≥ 3 fold, the non-responders had significantly more fully inactivating mutations of senescence-inducing cell cycle genes (*CDKN2A*/*B*/*C; CDKN1A*/*B; RB1; TP53; JAK1*/*2*/*3*) or ≥ 3 fold amplifications of genes promoting cell cycle progression (*CCND1*/*2*/*3; CDK4*/*6; CCNE1; MDM2*/*4; MYC*) than the responders (Fig. 4c; Fig. 13, 10). The genetic data were supported by functional analyses *in vitro,* revealing that melanoma lines from non-responders were resistant to CIS, but susceptible to apoptosis (Fig. 4d), while melanoma lines from responder patients were susceptible to both, CIS and apoptosis (Fig. 4d). While the inventors' findings support the established role of direct cancer cell rejection by the immune system, they demonstrate that cytotoxicity alone may be insufficient for cancer control. In consequence, cancer immune-control requires in addition IFN-dependent activation of cancer-intrinsic senescence-inducing cell cycle regulators, as a second mechanism to protect against those cancer cells that escape from cytotoxicity.

### 4. References

1. Mocikat, R. et al. Natural killer cells activated by MHC class I(low) targets prime dendritic cells to induce protective CD8 T cell responses. Immunity 19, 561-569 (2003).
2. Tumeh, P. C. et al. PD-1 blockade induces responses by inhibiting adaptive immune resistance. Nature 515, 568-571 (2014).
3. Twyman-Saint Victor, C. et al. Radiation and dual checkpoint blockade activate non-redundant immune mechanisms in cancer. Nature 520, 373-377 (2015).
4. Landsberg, J. et al. Melanomas resist T-cell therapy through inflammation-induced reversible dedifferentiation. Nature 490, 412-416 (2012).
5. Sagiv-Barfi, I. et al. Eradication of spontaneous malignancy by local immunotherapy. Sci. Transl. Med. 10 (2018).
6. Ribas, A. & Wolchok, J. D. Cancer immunotherapy using checkpoint blockade. Science 359, 1350-1355 (2018).
7. Pollard, J. W. Defining metastatic cell latency. N. Engl. J. Med. 375, 280-282 (2016).
8. Massague, J. & Obenauf, A. C. Metastatic colonization by circulating tumour cells. Nature 529 (2016).
9. Robinson, D. R. et al. Integrative clinical genomics of metastatic cancer. Nature 548, 297-303 (2017).
10. Braumüller, H. et al. T-helper-1-cell cytokines drive cancer into senescence. Nature 494, 361-365 (2013).
11. Patel, S. J. et al. Identification of essential genes for cancer immunotherapy. Nature 548, 537-542 (2017).
12. Pan, D. et al. A major chromatin regulator determines resistance of tumor cells to T cell-mediated killing. Science 359, 770-775 (2018).
13. Ferrari de Andrade, L. et al. Antibody-mediated inhibition of MICA and MICB shedding promotes NK cell-driven tumor immunity. Science 359, 1537-1542 (2018).
14. Schreiber, R. D., Old, L. J. & Smyth, M. J. Cancer immunoediting: integrating immunity's roles in cancer suppression and promotion. Science 331, 1565-1570 (2011).
15. Blank, C. U., Haanen, J. B., Ribas, A. & Schumacher, T. N. Cancer immunology. The "cancer immunogram". Science 352, 658-660 (2016).
16. Wieder, T., Eigentler, T., Brenner, E. & Rocken, M. Immune checkpoint blockade therapy. J. Allergy Clin. Immunol., S0091-6749(18)30445-7 (2018).
17. Topalian, S. L., Drake, C. G. & Pardoll, D. M. Immune checkpoint blockade: a common denominator approach to cancer therapy. Cancer Cell 27, 450-461 (2015).
18. Baumeister, S. H., Freeman, G. J., Dranoff, G. & Sharpe, A. H. Coinhibitory pathways in immunotherapy for cancer. Annu. Rev. Immunol. 34, 539-573 (2016).
19. Zaretsky, J. M. et al. Mutations associated with acquired resistance to PD-1 blockade in melanoma. N. Engl. J. Med. 375, 819-829 (2016).
20. Sucker, A. et al. Acquired IFNgamma resistance impairs anti-tumor immunity and gives rise to T-cell-resistant melanoma lesions. Nat Commun 8, 15440 (2017).
21. Manguso, R. T. et al. In vivo CRISPR screening identifies Ptpn2 as a cancer immunotherapy target. Nature 547, 413-418 (2017).
22. Sun, R. & Gao, B. Negative regulation of liver regeneration by innate immunity (natural killer cells/interferon-gamma). Gastroenterology 127, 1525-1539 (2004).
23. Kammertoens, T. et al. Tumour ischaemia by interferon-gamma resembles physiological blood vessel regression. Nature 545, 98-102 (2017).
24. Campisi, J. Aging, cellular senescence, and cancer. Annu. Rev. Physiol. 75, 685-705 (2013).
25. Sosa, M. S., Bragado, P. & Aguirre-Ghiso, J. A. Mechanisms of disseminated cancer cell dormancy: an awakening field. Nat. Rev. Cancer 14, 611-622 (2014).
26. Milanovic, M. et al. Senescence-associated reprogramming promotes cancer stemness. Nature 553, 96-100 (2018).
27. Perez-Mancera, P. A., Young, A. R. & Narita, M. Inside and out: the activities of senescence in cancer. Nat. Rev. Cancer 14, 547-558 (2014).
28. Serrano, M., Lin, A. W., McCurrach, M. E., Beach, D. & Lowe, S. W. Oncogenic ras provokes premature cell senescence associated with accumulation of p53 and p16INK4a. Cell 88, 593-602 (1997).
29. Goding, S. R. et al. Restoring immune function of tumor-specific CD4+ T cells during recurrence of melanoma. J. Immunol. 190, 4899-4909 (2013).
30. Ayers, M. et al. IFN-gamma-related mRNA profile predicts clinical response to PD-1 blockade. J. Clin. Invest. (2017).
31. Kovalchuk, A. L. et al. Burkitt lymphoma in the mouse. J. Exp. Med. 192, 1183-1190 (2000).
32. Forster, I., Hirose, R., Arbeit, J. M., Clausen, B. E. & Hanahan, D. Limited capacity for tolerization of CD4+ T cells specific for a pancreatic beta cell neo-antigen. Immunity 2, 573-585 (1995).
33. Bergers, G., Javaherian, K., Lo, K. M., Folkman, J. & Hanahan, D. Effects of angio-genesis inhibitors on multistage carcinogenesis in mice. Science 284, 808-812 (1999).
34. Meraz, M. A. et al. Targeted disruption of the Stat1 gene in mice reveals unexpected physiologic specificity in the JAK-STAT signaling pathway. Cell 84, 431-442 (1996).
35. Müller-Hermelink, N. et al. TNFR1 signaling and IFN-gamma signaling determine whether T cells induce tumor dormancy or promote multistage carcinogenesis. Cancer Cell 13, 507-518 (2008).
36. Naito, Y., Hino, K., Bono, H. & Ui-Tei, K. CRISPRdirect: software for designing CRISPR/Cas guide RNA with reduced off-target sites. Bioinformatics 31, 1120-1123 (2015).
37. Ran, F. A. et al. Genome engineering using the CRISPR-Cas9 system. Nat. Protoc. 8, 2281-2308 (2013).
38. Krepler, C. et al. Personalized Preclinical Trials in BRAF Inhibitor-Resistant Patient-Derived Xenograft Models Identify Second-Line Combination Therapies. Clin. Cancer Res. 22, 1592-1602 (2016).
39. Lasithiotakis, K. G. et al. Combined inhibition of MAPK and mTOR signaling inhibits growth, induces cell death, and abrogates invasive growth of melanoma cells. J. Invest. Dermatol. 128, 2013-2023 (2008).
40. Schmid, A., Braumüller, H., Wehrl, H. F., Rocken, M. & Pichler, B. J. Non-invasive monitoring of pancreatic tumor progression in the RIP1-Tag2 mouse by magnetic resonance imaging. Mol. Imaging Biol. 15, 186-193 (2013).
41. Brede, C. et al. Mapping immune processes in intact tissues at cellular resolution. J. Clin. Invest. 122 (2012).
42. Vandesompele, J. et al. Accurate normalization of real-time quantitative RT-PCR data by geometric averaging of multiple internal control genes. Genome Biol. 3, 1-12 (2002).
43. Li, H. & Durbin, R. Fast and accurate short read alignment with Burrows-Wheeler transform. Bioinformatics 25, 1754-1760 (2009).
44. Kim, S. et al. Strelka2: fast and accurate calling of germline and somatic variants. Nat Methods 15, 591-594 (2018).
45. Cingolani, P. et al. A program for annotating and predicting the effects of single nucleotide polymorphisms, SnpEff: SNPs in the genome of Drosophila melanogaster strain w1118; iso-2; iso-3. Fly (Austin) 6, 80-92 (2012).
46. Johnson, D. B. et al. Targeted next generation sequencing identifies markers of response to PD-1 blockade. Cancer Immunol Res 4, 959-967 (2016).
47. Schroeder, C. M. et al. A comprehensive quality control workflow for paired tumor-normal NGS experiments. Bioinformatics 33, 1721-1722 (2017).

## Claims

1. A method ex vivo for classifying a patient in need as non-responder or responder to immune checkpoint inhibitor therapy, comprising the following steps:
1) providing a biological sample originating from said patient;
2) analyzing the biological sample for the presence or absence of a genetic alteration causing a modification of function in
- cellular senescence-inducing cell cycle regulator genes, and/or
- cellular senescence-inhibiting cell cycle regulator genes,
3) classifying the patient as
- non-responder if in step (2) said genetic alterations are identified as present, wherein said cellular senescence-inhibiting cell cycle regulator genes are selected from the group consisting of: *MDM2, MDM4, CDK4, CCND1, CCND2, CCND3, CDKN1B, CDK6, CCNE1, RB1, E2F2,* and *MYC.*

2. The method of claim 1, wherein said genetic alteration causing a modification of function is a
- loss-of-function mutation in cellular senescence-inducing cell cycle regulator genes, and/or
- gain-of-function mutation in cellular senescence-inhibiting cell cycle regulator genes.

3. The method of claim 1 or 2, wherein said cellular senescence-inducing cell cycle regulator genes are selected from the group consisting of: CDKN2B, CDKN2C, CDKN1A, CDKN1B, RB1, TP53, STAT1, JAK1, JAK2, JAK3, and CDKN2A.

4. The method of any of the preceding claims, wherein in step (3) said classification as a non-responder occurs if a genetic modification is identified as present in at least 1, or at least 2, or at least 3, or at least 4, or at least 5, or at least 6, or at least 7, or at least 8, or at least 9, or at least 10, or at least 11 or at least 12 different cellular senescence-inducing cell cycle regulator genes and/or senescence-inhibiting cell cycle regulator genes.

5. The method of any of the preceding claims, wherein said patient is suffering from a melanoma, preferably a metastatic melanoma.

6. The method of any of the preceding claims, wherein said patient is suffering from a carcinoma, preferably a metastatic carcinoma.

7. The method of any of the preceding claims, wherein said patient is suffering from a lymphoma.

8. Use of a gene set comprising (i) cellular senescence-inducing cell cycle regulator genes, and (ii) cellular senescence-inhibiting cell cycle regulator genes for classifying a patient in need as non-responder or responder to immune checkpoint inhibitor therapy, wherein said cellular senescence-inhibiting cell cycle regulator genes are selected from the group consisting of: MDM2, MDM4, CDK4, CCND1, CCND2, CCND3, CDK6, CCNE1, E2F2, and MYC.

9. Use of claim 8, wherein said cellular senescence-inducing cell cycle regulator genes are selected from the group consisting of: CDKN2B, CDKN2C, CDKN1A, CDKN1B, RB1, TP53, STAT1, JAK1, JAK2, JAK3, and CDKN2A.

10. Use of claims 8 or 9, wherein said gene set comprises at least 1, preferably at least 2, further preferably at least 3, further preferably at least 4, further preferably at least 5, further preferably at least 6, further preferably at least 7, further preferably at least 8, further preferably at least 9, further preferably at least 10, highly preferably at least11 different cellular senescence-inducing cell cycle regulator genes and/or senescence-inhibiting cell cycle regulator genes.
